(19) Europäisches Patentamt
European Patent Office
Office européen des brevets

(11) Veröffentlichungsnummer: 0 144 459
A1

# (12) EUROPÄISCHE PATENTANMELDUNG

(21) Anmeldenummer: 83112409.4

(22) Anmeldetag: 09.12.83

(51) Int. Cl.[4]: C 07 C 177/00
A 61 K 31/557

(43) Veröffentlichungstag der Anmeldung: 19.06.85 Patentblatt 85/25

(84) Benannte Vertragsstaaten: AT BE CH DE FR GB IT LI LU NL SE

(71) Anmelder: Dr. Willmar Schwabe GmbH & Co.
Willmar-Schwabe-Strasse 4
D-7500 Karlsruhe 41(DE)

(72) Erfinder: Weinges, Klaus, Prof.Dr.Dipl.Chem
Langgewann 41
D-6900 Heidelberg(DE)

(74) Vertreter: Bunke, Holger, Dr.rer.nat. Dipl.-Chem. et al,
Patentanwälte Prinz, Leiser, Bunke & Partner
Ernsbergerstrasse 19
D-8000 München 60(DE)

## (54) Prostaglandine, Verfahren zu ihrer Herstellung und diese enthaltende pharmazeutische Zubereitungen.

(57) Die Erfindung betrifft Prostaglandine des Typs 12-epi-$PGF_{2\beta}$, insbesondere 12-epi-$PGF_{2\beta}$ und 15-Methyl-12-epi-$PGF_{2\beta}$, deren Niedrigalkylester sowie deren 15R-Epimere. Außerdem wird ein neuer, einfacher und stereoselektiver Zugang zu den Prostaglandinen des Typs $F_{2\beta}$ und der 12-epi-Reihe dieses Typs vorgeschlagen. Die Erfindung betrifft schließlich pharmazeutige Zubereitungen, die mindestens eine der erfindungsgemäßen Verbindungen enthalten und die zur Behandlung vaskulärer Erkrankungen und zur Therapie von Erkrankungen, die bisher mit Prostaglandinen vom $F_{2\alpha}$-Typus behandelt wurden, verwendet werden können.

EP 0 144 459 A1

PRINZ, LEISER, BUNKE & PARTNER
Patentanwälte European Patent Attorneys
München Stuttgart

0144459



Dr. Willmar Schwabe GmbH & Co.
Willmar-Schwabe-Straße 4
7500 Karlsruhe 41

9. Dezember 1983

Unser Zeichen: P 2627 EP

---

Prostaglandine, Verfahren zu ihrer Herstellung und diese enthaltende pharmazeutische Zubereitungen

---

Die Erfindung betrifft Prostaglandine der $F_2$-Reihe ($PGF_2$), Verfahren zu ihrer Herstellung und diese enthaltende pharmazeutische Zubereitungen. (Zur Kennzeichnung der PG-Reihen, zur Nomenklatur und Bezifferung vgl.: Ullmann's Enzyklopädie der Techn. Chemie, 4. Aufl., Bd. 19, S. 483 ff. (1980); H. König in: Klinische Wochenschrift 53, 1041-1048 (1975)).

Prostaglandine sind im tierischen und menschlichen Organismus ubiquitär verbreitet. Die Vielfalt ihrer pharmakologischen Wirkungen erklärt das anhaltende Interesse, aber auch das Bedürfnis an der Erforschung natürlicher und synthetischer Prostaglandin-Derivate. Die Prostaglandine werden als lokale Modulatoren und Regulatoren hormonaler Effekte auf die Zellfunktion angesehen. Ihre bedeutendsten Wirkungen sind, soweit bisher bekannt, die abortive und luteolytische Wirkung, die blutdruckregu-

Bj/Ma

licrende Wirkung, die teils aggregierende, teils die Aggregation hemmende Wirkung auf die Blutplättchen, die Beeinflussung der Magensaftsekretion und die bronchospasmolytische Wirkung (vgl. z.B. S. Bergström, Angew. Chem. 95, 865 (1983)).

Die bisherigen Forschungsarbeiten auf dem Gebiet der Prostaglandine haben gezeigt, daß die pharmakologischen Wirkungen in besonders hohem Maße von der sterischen Anordnung der Substituenten am Cyclopentanring der von der Prostansäure abgeleiteten Verbindungen abhängen. Es ist deshalb selbst für den Fachmann nicht vorhersehbar, in welcher Weise sich die biologische Aktivität und das Wirkungsspektrum der Prostaglandin-Derivate ändern, wenn die Konformation der Verbindungen auch nur an einem Chiralitätszentrum geändert wird.

Die F-Reihe der natürlichen Prostaglandine besitzt am Cyclopentanring folgende Konfiguration:

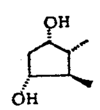

F

Zu dieser Reihe gehört das in der Natur vorkommende $PGF_{2\alpha}$, also die 9α,11α,15S-Trihydroxy-5-cis,13-trans-prostadiensäure:

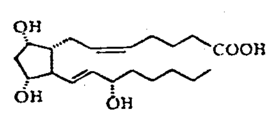

$PGF_{2\alpha}$

Aus der DE-OS 23 60 893 sind auch bereits 12-epi-Derivate

der Prostaglandine der F-Reihe und ein Verfahren zu ihrer Herstellung bekannt, insbesondere das 12-epi-$PGF_{2\alpha}$, das sich vom natürlichen $PGF_{2\alpha}$ nur dadurch unterscheidet, daß die Alkylseitenkette an $C^{12}$ nicht mehr ß-ständig, sondern ebenfalls α-ständig ist, also bezüglich der durch den Cyclopentanring verlaufenden Ebene auf derselben Seite sich befindet wie die Carboxylkette an $C^{8}$ und die beiden Hydroxygruppen an $C^{9}$ und $C^{11}$.

Das aus der DE-OS 23 60 893 bekannte Verfahren zur stereoselektiven Herstellung des 12-epi-$PGF_{2\alpha}$ und analoger 12-epi-Derivate geht von 7-Acetoxymethylen-5-chloro-5-cyanobicyclo [ 2.2.1 ] hept -2-en aus, das in sechs Verfahrensstufen zu 2,3,3aß,6aß-tetrahydro-5 α -hydroxy-4 α -(3-hydroxy-4-m-chlorophenoxy-but-1-trans-enyl)-6ß-iodo-2-oxocyclopenteno[b] furan umgesetzt, in drei weiteren Verfahrensstufen jedoch nicht stereoselektiv, sondern in Form von Epimerengemischen bis zum 2-Hydroxy-2,3,3 αß, 6αß-tetrahydro-5 α -(tetrahydropyran-2-yloxy)-4α-[3-(tetrahydropyran-2-yloxy)-4-m-chlorophenoxybut-1-trans-enyl] -cyclopenteno [ b ] furan umgesetzt wird, welches dann in die 16-(3-Chlorophenoxy)-9 α -hydroxy-11 α ,15-bis(tetrahydropyran-2-yloxy)-17,18,19,20-tetranor-5-cis,13-trans-12-epi-prostadiensäure übergeführt werden kann. Aus letzterem werden dann die beiden $C^{15}$-Epimeren des 12-epi-$PGF_{2\alpha}$ hergestellt (vgl. a.a.O., Beispiel 5), die aber offenbar nicht einzeln isoliert und nicht eindeutig charakterisiert werden konnten.

Außer dem $PGF_{2\alpha}$ und dem 12-epi-$PGF_{2\alpha}$ ist auch bereits das $PGF_{2\beta}$ bekannt (z.B. aus der GB-PS 1 332 262), welches das $C^{9}$-Epimere des $PGF_{2\alpha}$ darstellt.

Der Erfindung liegt die Aufgabe zugrunde, Prostaglandine des Typs 12-epi-$PGF_{2\beta}$ der allgemeinen Formel I

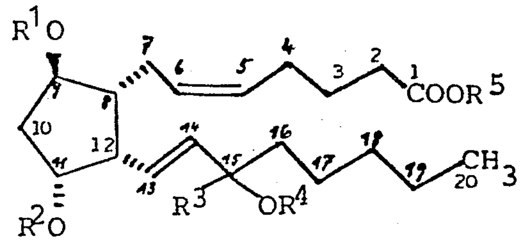

(I)

und ein möglichst einfaches, jedoch stereoselektives Verfahren zu ihrer Herstellung sowie ein Verfahren zur Herstellung von $PGF_{2\beta}$ der Formel VI

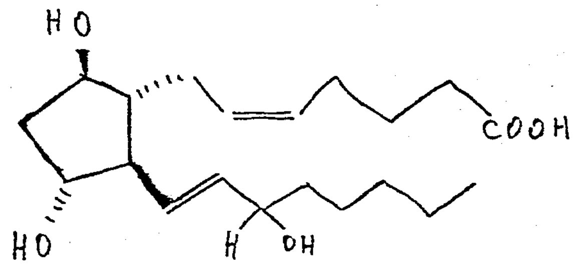

(VI)

und damit überhaupt einen neuen und einfachen Zugang zu den 12-epi-Prostaglandinen und zu $PGF_{2\beta}$ zu schaffen, um so neue Prostaglandin-Pharmaka bereitzustellen und neue Wege zur Synthese pharmakologisch wirksamer Prostaglandin-Derivate zu eröffnen.

Diese Aufgabe wird durch die Bereitstellung der erfindungsgemäßen Verbindungen, durch die erfindungsgemäßen Verfahren und die erfindungsgemäßen pharmazeutischen Zubereitungen gelöst.

Die erfindungsgemäßen Verbindungen sind Prostaglandine des Typs 12-epi-$PGF_{2\beta}$ der allgemeinen Formel I, worin

$R^1$ und $R^2$, die verschieden oder gleich sein können, ein Wasserstoffatom, eine Benzylgruppe, eine aliphatische Acylgruppe mit 2-5 C-Atomen, eine aromatische Acylgruppe mit 7-13 C-Atomen oder eine andere bekannte Schutzgruppe,

$R^3$ ein Wasserstoffatom, eine Methyl-, Ethyl- oder Isopropylgruppe,

$R^4$ ein Wasserstoffatom, eine Tetrahydropyranylgruppe oder eine andere bekannte Schutzgruppe und

$R^5$ ein Wasserstoffatom, eine geradkettige oder verzweigte Alkylgruppe mit 1-4 C-Atomen oder eine bekannte Schutzgruppe

bedeuten, sowie deren physiologisch unbedenkliche Salze.

Beispiele für aliphatische Acylgruppen mit 2-5 C-Atomen sind Acetyl, Propionyl, Isobutyryl und Beispiele für aromatische Acylgruppen mit 7-13 C-Atomen sind Benzoyl, p-Nitrobenzoyl, p-Phenylbenzoyl.

Unter dem Ausdruck "eine andere bekannte Schutzgruppe" ist jede Schutzgruppe zu verstehen, die für den Schutz von O-H-Bindungen bekannt und dem Fachmann geläufig ist. Diese Schutzgruppen sind beispielsweise beschrieben von J.F.W. McOmie in Adv. in Org. Chem. 3, 216-223 (1963).

Zu den erfindungsgemäßen Verbindungen gehören sowohl die $C^{15}$-Epimerengemische als auch, vorzugsweise, die isolierten 15S- und 15R-Epimeren.

Besonders bevorzugte erfindungsgemäße Verbindungen sind die $C^{15}$-Epimeren des 12-epi-$PGF_{2\beta}$ und des 15-Methyl-12-epi-$PGF_{2\beta}$ sowie deren Niedrigalkylester, insbesondere Methylester, gemäß den Patentansprüchen 4 bis 15.

Eine erste Variante des erfindungsgemäßen Verfahrens zur Herstellung der 12-epi-Prostaglandine der allgemeinen Formel I ist durch die Kombination folgender Merkmale gekennzeichnet:

(a) eine Verbindung der allgemeinen Formel II,

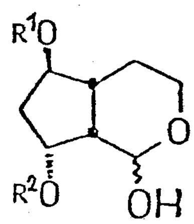

(II)

worin $R^1$ und $R^2$ die in Anspruch 1 genannten Bedeutungen, ausgenommen $R^1 = R^2 = H$, haben, wird mit einem geeigneten Wittig-Horner-Reagens zur entsprechenden Verbindung der allgemeinen Formel III,

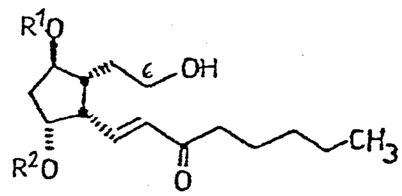

(III)

worin $R^1$ und $R^2$ die in Anspruch 1 genannten Bedeutungen, ausgenommen $R^1 = R^2 = H$, haben, umgesetzt;

(b) die 6-Hydroxygruppe der Verbindung der allgemeinen Formel III wird durch Einführung einer Schutzgruppe geschützt;

(c) die 15-Ketogruppe wird unter Bildung des 15S/15R-Epimerengemischs der entsprechenden Verbindung der allgemeinen Formel IV,

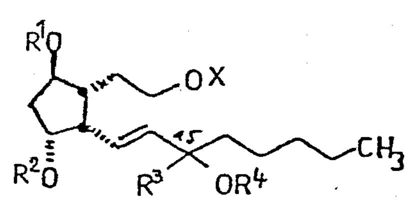

(IV)

worin $R^4$ ein Wasserstoffatom und X die Schutzgruppe ist, umgesetzt, und zwar für $R^3$ = H mit einem Reduktionsmittel und für $R^3$ = $C_1$- bis $C_3$-Alkyl mit einer metallorganischen Verbindung mit 1-3 C-Atomen, wonach die 15S- und 15R-Epimeren chromatographisch voneinander getrennt werden;

(d) die 15-Hydroxygruppe der Epimeren der allgemeinen Formel IV wird durch Einführung einer weiteren Schutzgruppe $R^4$ geschützt, wonach die Schutzgruppe X oxidativ abgespalten wird unter Bildung der entsprechenden Verbindung der allgemeinen Formel V,

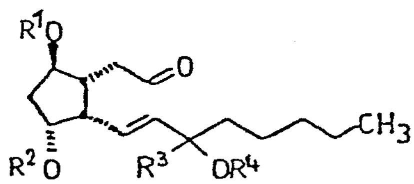

(V)

worin $R^1$, $R^2$, $R^3$ und $R^4$ die in Anspruch 1 genannten Bedeutungen, ausgenommen $R^1$, $R^2$, $R^4$ = H, haben;

(e) die Verbindung der allgemeinen Formel V wird mit einem geeigneten Pentansäure-Ylid zur entsprechenden Verbindung der allgemeinen Formel I umgesetzt, worin $R^1$, $R^2$, $R^3$ und $R^4$ die in Anspruch 1 genannten Bedeutungen, ausgenommen $R^1$, $R^2$, $R^4$ = H, haben und worin $R^5$ = H ist;

(f) wonach die Schutzgruppen $R^1$, $R^2$ und $R^4$ gegebenenfalls wieder abgespalten und/oder die freie Säure in ein physiologisch unbedenkliches Salz übergeführt oder verestert wird.

Eine zweite Variante des erfindungsgemäßen Verfahrens zur Herstellung der 12-epi-Prostaglandine der allgemeinen Formel I und des $PGF_{2\beta}$ geht wiederum von einer Verbindung der allgemeinen Formel II aus, worin $R^1$ und $R^2$ die in

 

Anspruch 1 genannten Bedeutungen, ausgenommen $R^1 = R^2 = H$, haben.

Die Verbindung der Formel II wird

(a) mit einem geeigneten Reduktionsmittel zu einem Diol der allgemeinen Formel VII reduziert,

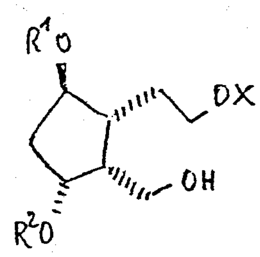

(VII)

worin $R^1$ und $R^2$ die in Anspruch 1 genannten Bedeutungen, ausgenommen $R^1 = R^2 = H$, haben und X = H ist;

(b) wonach das Diol mit einem reaktiven Säurederivat zur entsprechenden Verbindung der allgemeinen Formel VII, worin X der Acylrest des reaktiven Säurederivats ist, verestert wird;

(c) dann die verbliebene Hydroxymethylgruppe zur Aldehydgruppe unter Bildung der entsprechenden Verbindung der allgemeinen Formel VIII oxidiert wird,

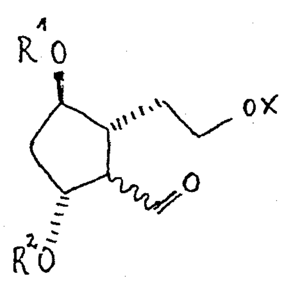

(VIII)

(d) wonach die Verbindung der allgemeinen Formel VIII mit einem geeigneten Wittig-Horner-Reagens zum $C^{12}$-Epimerengemisch der allgemeinen Formel IX,

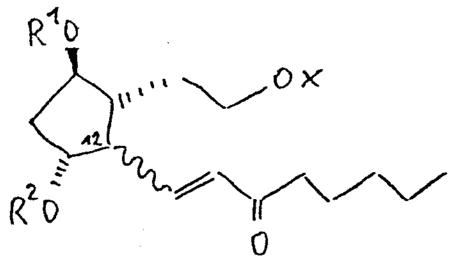

(IX)

worin $R^1$ und $R^2$ die in Anspruch 1 genannten Bedeutungen, ausgenommen $R^1 = R^2 = H$, haben und X die Acylschutzgruppe ist, umgesetzt wird, worauf die $C^{12}$-Epimeren säulenchromatographisch voneinander getrennt werden;

(e) worauf schließlich das 12α - Epimere entsprechend der ersten Variante, Abschnitte (c) bis (f), des Verfahrens zu den erfindungsgemäßen 12-epi-Prostaglandinen umgesetzt wird,

(f) während das 12β-Epimere der allgemeinen Formel IX unter Bildung des 15S/15R-Epimerengemischs der entsprechenden Verbindung der allgemeinen Formel X,

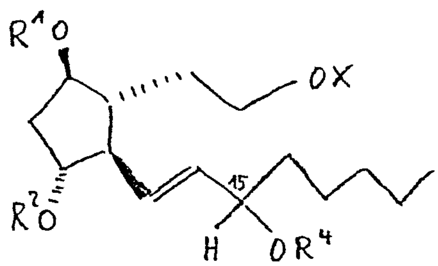

(X)

worin $R^4$ ein Wasserstoffatom ist, reduziert wird, wonach die $C^{15}$-Epimeren chromatographisch voneinander getrennt werden;

(g) die Verbindung der allgemeinen Formel X wird entsprechend der ersten Variante, Abschnitte (d) bis (f), des Verfahrens zum $PGF_{2\beta}$ (Formel VI) umgesetzt.

Vorteilhafte Ausführungsbeispiele des erfindungsgemäßen Verfahrens bestehen darin, daß als Wittig-Horner-Reagens 2-Oxoheptylphosphonsäure-dimethylester, als Schutzgruppe X der p-Phenylbenzoylrest, als metallorganische Verbindung mit 1-3 C-Atomen eine entsprechende Grignard-Verbindung wie Methylmagnesiumiodid, Ethylmagnesiumbromid, Isopropylmagnesiumbromid oder Isopropylmagnesiumchlorid und als Pentansäure-Ylid das aus 5-Triphenylphosphoniumpentansäurebromid und Kalium-tert.-butylat gebildete Ylid verwendet werden.

Das erfindungsgemäße Verfahren geht somit aus von den Verbindungen der allgemeinen Formel II, die sich aus dem bekannten (6R,7R)-(-)-7-Hydroxy-3-oxabicyclo[4.3.0] non-1-en-9-on(1), welches wiederum aus dem Naturstoff Catalpol herstellbar ist, in guter Ausbeute synthetisieren lassen (vgl. K. Weinges et al., Angew. Chem. 92, 639-640 (1980), K. Weinges et al., Liebigs Ann. Chem. 1982, 872, und DE-OS 30 30 477).

Vorzugsweise sind die beiden Reste R in der Verbindung der allgemeinen Formel II gleich; sie können aber auch verschieden sein. Die Reste R können aliphatische Acylgruppen mit 2-5 C-Atomen, aromatische Acylgruppen mit 7-13 C-Atomen oder andere bekannte Schutzgruppen im Sinne der eingangs erwähnten Definition wie zum Beispiel die Benzylgruppe sein.

Zur weiteren Erläuterung des erfindungsgemäßen Verfahrens wird zunächst mit Hilfe des Formelschemas 1 in Verbindung mit den Beispielen 1a bis 1c die Bereitstellung der Ausgangsverbindungen (2) der allgemeinen Formel II beschrieben.

Die Beispiele 1a und 1b betreffen die Synthese von 2 mit zwei gleichen Schutzgruppen ($R^1 = R^2$), und zwar mit Benzylgruppen und mit p-Phenylbenzoylgruppen, während das Beispiel 1c die Darstellung einer Verbindung 2 beschreibt, die zwei verschiedene Schutzgruppen ($R^1 \neq R^2$) enthält (einen p-Nitrobenzoylrest und eine beliebige Schutzgruppe, z.B. den Tetrahydropyranylrest).

<u>Formelschema 1</u>

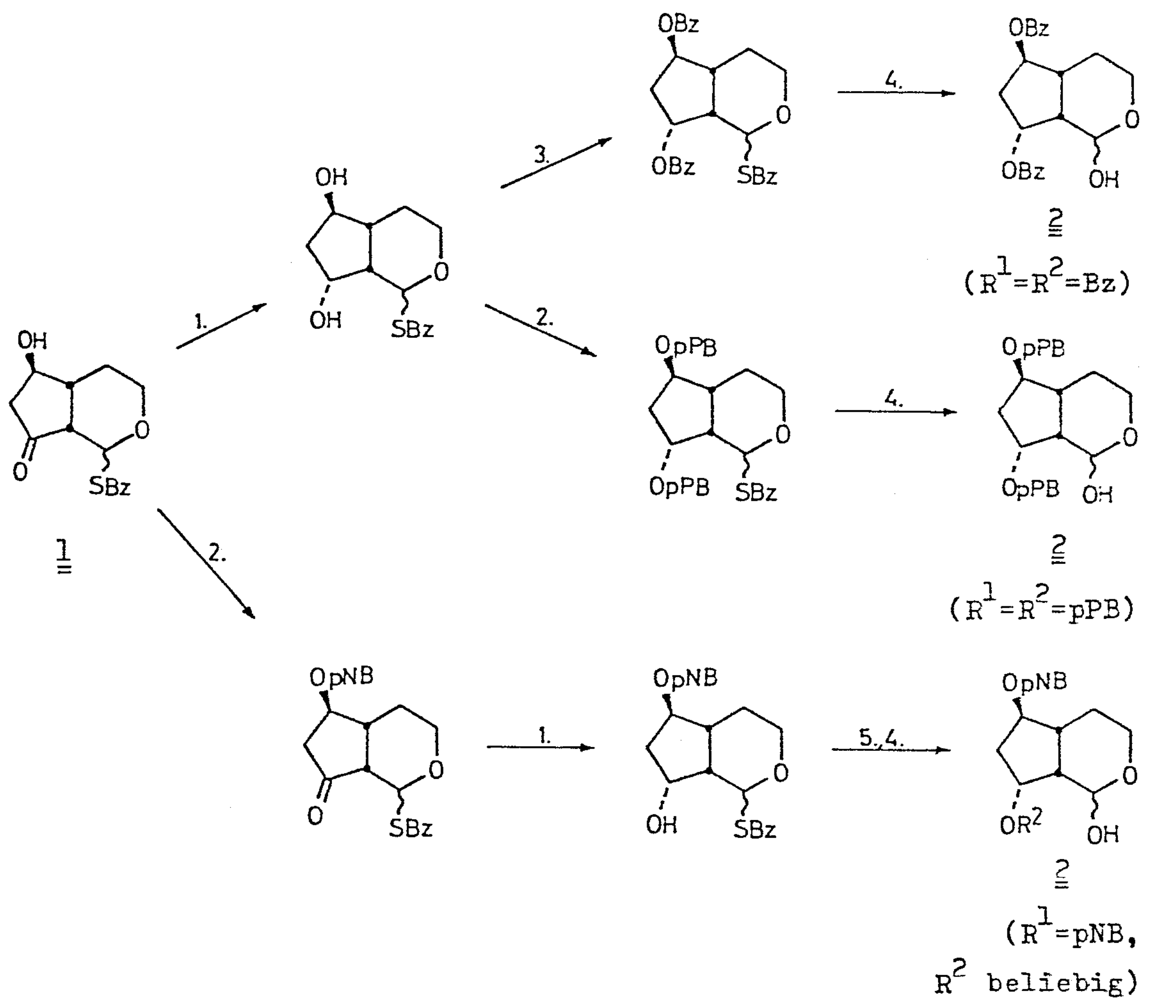


1

2 ($R^1=R^2=Bz$)

2 ($R^1=R^2=pPB$)

2 ($R^1=pNB$, $R^2$ beliebig)

Reaktionsstufen

1. Reduktion, Epimerentrennung
2. Veresterung
3. Veretherung
4. Reaktion mit $Hg^{2+}$
5. Einführung einer Schutzgruppe $R^2$

Bz = Benzyl, pPB = Phenylbenzoyl, pNB = p-Nitrobenzoyl

In Beispiel 2 (Formelschema 2) wird von derjenigen Verbindung der allgemeinen Formel II ausgegangen, worin $R^1$ und $R^2$ jeweils eine Benzylgruppe bedeuten, also vom (1S,6R,7R,9R)-(-)-7,9-Dibenzyloxy-2-hydroxy-3-oxabicyclo-[4.3.0]nonan (2, $R^1$ = $R^2$ = Benzyl).

Der überraschende Vorteil dieser Variante des erfindungsgemäßen Verfahrens besteht darin, daß der stereoselektive Aufbau der Alkyl-Seitenkette an $C^{12}$ bereits in der ersten Stufe des vielstufigen Verfahrens erfolgt, wodurch die 12-epi-Konfiguration bereits für den gesamten Syntheseweg festgelegt wird. Diese erste Stufe ist eine Wittig-Horner-Reaktion mit einem geeigneten, also bereits einen Heptylrest enthaltenden Wittig-Horner-Reagens. Vorzugsweise wird hierzu 2-Oxoheptylphosphonsäure-dimethylester verwendet, bei dessen Umsetzung mit Verbindung 2 überraschenderweise keine Epimerisierung am $C^1$-, dem späteren $C^{12}$-Atom stattfindet, wie an sich zu erwarten gewesen wäre (vgl. W.F.Berkowitz et al., J.Org. Chem. 47, 824 (1982)), sondern stereoselektiv nur das "cis"-Isomere 3 entsteht. Daher kann 2 zur Herstellung verschiedener enantiomerenreiner 12-epi-Prostaglandine verwendet werden.

In der zweiten Stufe wird die primäre Hydroxylgruppe von 3 wird durch Einführung einer geeigneten Schutzgruppe geschützt; vorzugsweise wird 3 mit p-Phenylbenzoylchlorid umgesetzt. Mit dem p-Phenylbenzoylrest als Schutzgruppe läßt sich nämlich nach der Reduktion der Carbonylgruppe an $C^{15}$ eine quantitative Trennung von 5 in die 15S- und 15R-Epimeren, die im Verhältnis 2:3 erhalten werden, durchführen. Die Reduktion von 4 zu 5 kann beispielsweise mit frisch hergestelltem Zinkboranat in Dimethoxyäthan durchgeführt werden. Das bei der Reduktion entstehende Diastereomerengemisch wird chromatographisch, vorzugsweise säulenchromatographisch, in die beiden Epimeren 5 und (15R)-5 getrennt.

Formelschema 2

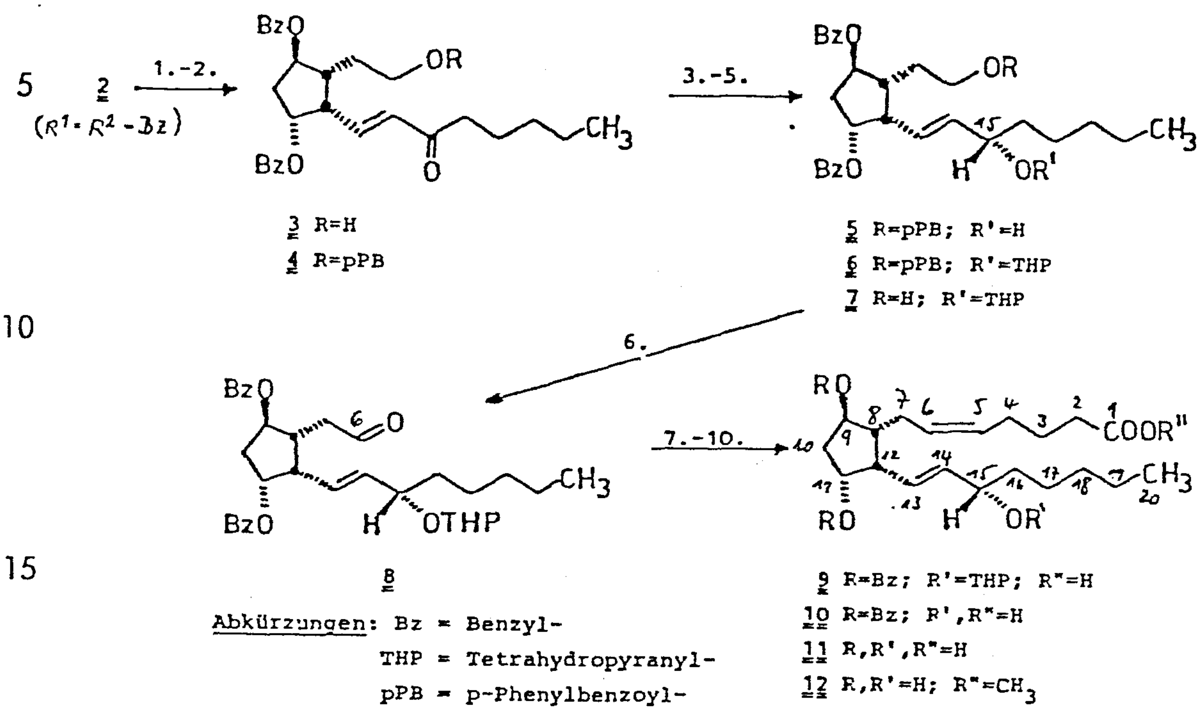


2 ($R^1 = R^2 = Bz$)

3 R=H
4 R=pPB

5 R=pPB; R'=H
6 R=pPB; R'=THP
7 R=H; R'=THP

8

9 R=Bz; R'=THP; R"=H
10 R=Bz; R',R"=H
11 R,R',R"=H
12 R,R'=H; R"=$CH_3$

Abkürzungen: Bz = Benzyl-
THP = Tetrahydropyranyl-
pPB = p-Phenylbenzoyl-

Reaktionsstufen:

1. Wittig-Horner-Reaktion (Alkylkette)
2. Schutz der primären OH-Gruppe
3. Reduktion der Carbonylgruppe
4. Schutz der $C^{15}$-OH-Gruppe
5. Hydrolytische Abspaltung der Schutzgruppe für die primäre OH-Gruppe an $C^6$
6. Oxidation der primären OH-Gruppe an $C^6$
7. Wittig-Reaktion (Carboxylkette)
8. Hydrolytische Abspaltung der Schutzgruppe für die $C^{15}$-OH-Gruppe
9. Abspaltung der Benzyl-Schutzgruppen an $C^9$ und $C^{11}$
10. Ggf. Veresterung

Je nachdem, ob als Endprodukt ein 15S- oder ein 15R-Derivat gewünscht wird, wird für die weiteren Umsetzungen 5 oder (15R)-5 verwendet.

In der nächsten Stufe wird die freie Hydroxylgruppe an $C^{15}$ durch Einführen einer Schutzgruppe, vorzugsweise durch eine Tetrahydropyranylgruppe, beispielsweise durch Umsetzen von 5 bzw. (15R)-5 mit Dihydropyran in saurer Lösung, geschützt. Aus der hierbei entstehenden Verbindung 6 wird in der nächsten Stufe die in der zweiten Stufe eingeführte Schutzgruppe für die primäre Hydroxylgruppe hydrolytisch abgespalten, wobei 7 entsteht.

Die freie Hydroxylgruppe in 7 wird anschließend zum Aldehyd 8 oxidiert, beispielsweise mit Pyridiniumchlorochromat.

In der nächsten Stufe wird schließlich die Carboxylkette des Prostaglandinderivats aufgebaut, und zwar 5-cisstereoselektiv durch Umsetzen von 8 mit einem geeigneten Pentansäure-Ylid, vorzugsweise mit dem aus 5-Triphenylphosphoniumpentansäurebromid und Kalium-tert.-butylat gebildeten Wittig-Reagens, zu 9, welches nach Abspaltung der Schutzgruppen, nämlich des Tetrahydropyranylrestes an $C^{15}$ und der beiden Benzylgruppen an $C^9$ und $C^{11}$, das gewünschte 12-epi-$PGF_{2\beta}$(11) ergibt. Nach säulenchromatographischer Reinigung fällt 11 als farbloses Öl an.

Der 12-epi-$PGF_{2\beta}$-methylester (12) kann aus 11 beispielsweise durch Umsetzung mit ätherischer Diazomethanlösung hergestellt werden. Auch 12 fällt als farbloses Öl an. Zur besseren Charakterisierung wird der kristalline 9,11,15-Tris(p-phenylbenzoyl)-12-epi-$PGF_{2\beta}$-methylester hergestellt.

Die Ausbeute an 11 beträgt 13%, bezogen auf 2. Auf dem

gleichen Weg wird aus (15R)-5 das kristalline 15R-12-epi-$PGF_{2\beta}$ (15R-11) in einer Ausbeute von 20%, bezogen auf 2, synthetisiert.

In Beispiel 3 (Formelschema 3) verwendet man dieselbe Ausgangsverbindung der allgemeinen Formel II, nämlich 2, $R^1 = R^2$ = Benzyl.

<u>Formelschema 3</u>

2 ($R^1 = R^2$ = Bz)

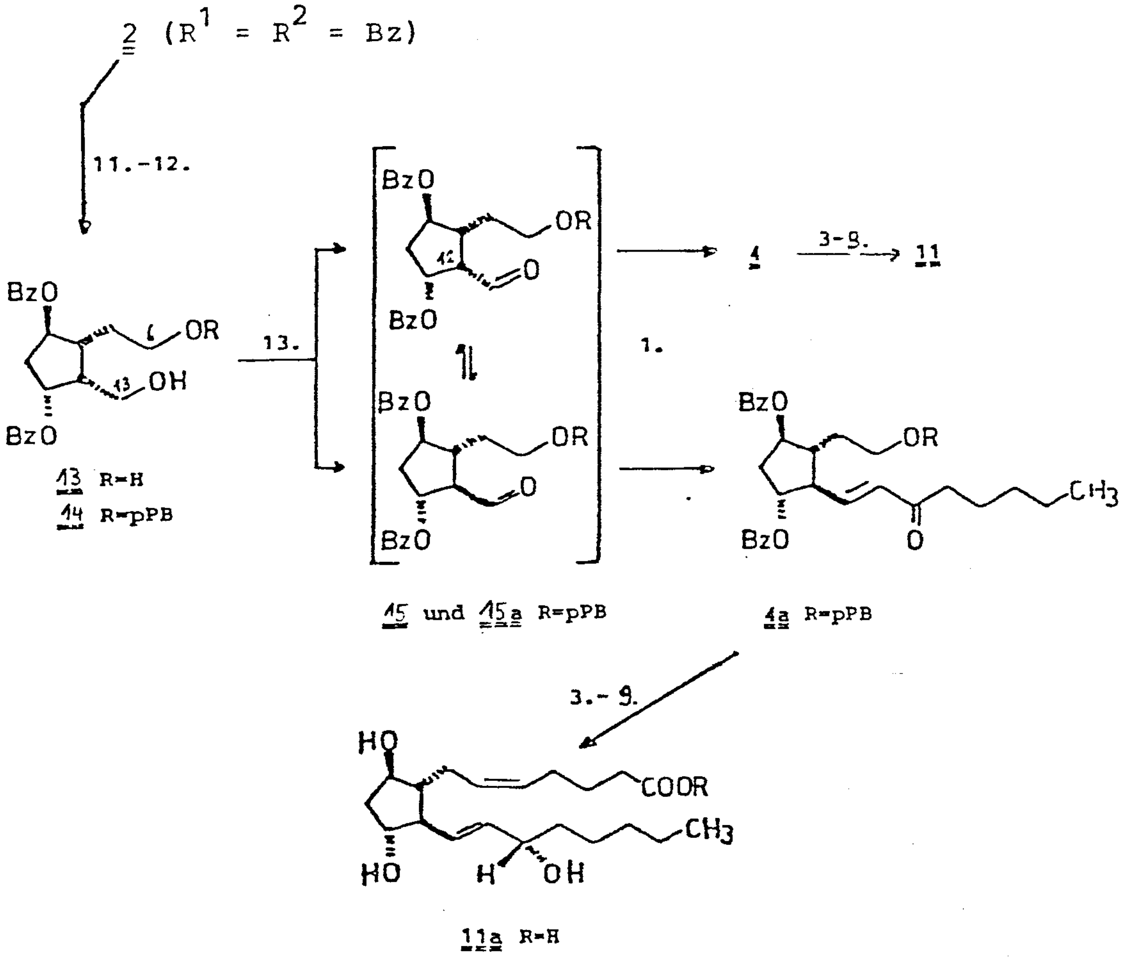


11. Reduktion des Lactols

12. Veresterung der OH-Gruppe an $C^6$

13. Oxidation der primären OH-Gruppe an $C^{13}$

1., 3. - 9. siehe Formelschema 2

2 wird in diesem Beispiel nicht mit dem Wittig-Horner-Reagens umgesetzt, sondern zu 13 reduziert, z.B. mit Lithiumaluminiumhydrid.

Das Diol 13 wird nun derart verestert, beispielsweise mit p-Phenylbenzoylchlorid, daß bevorzugt die OH-Gruppe an $C^{13}$ frei bleibt. Dennoch entstehender isomerer Ester und Diester werden wieder verseift, so daß 13 vollständig zu 14 umgesetzt wird.

Der Alkohol 14 wird in der nächsten Stufe oxidiert, beispielsweise mit Pyridiniumchlorochromat; der dabei entstehende Aldehyd liegt als Epimerengemisch 15/15a vor.

Dieses Epimerengemisch wird nun mit dem bereits in Beispiel 2 genannten Wittig-Horner-Reagens, vorzugsweise mit 2-Oxoheptylphosphonsäure-dimethylester, umgesetzt; die beiden entstehenden $C^{12}$-Epimere 4 und 4a werden getrennt, vorzugsweise säulenchromatographisch.

Das Epimere 4 wird gemäß Beispiel 2 zu den Prostaglandinen der 12-epi-Reihe weiter umgesetzt.

Das Epimere 4a (trans) wird analog dazu zum bekannten, kristallinen $PGF_{2\beta}$ (11a) umgesetzt. Die Ausbeute an 11a, bezogen auf 14, beträgt 8%.

In Beispiel 4 (Formelschema 4) wird von einer Verbindung der allgemeinen Formel II mit zwei p-Phenylbenzoylresten als Schutzgruppen ausgegangen (2,$R^1$=$R^2$=R=p-Phenylbenzoyl).

Formelschema 4

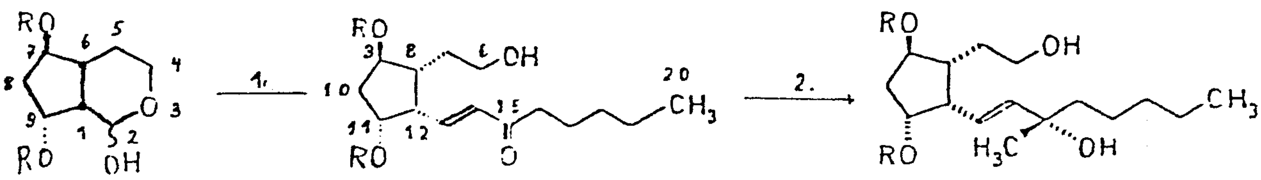


2 16 17

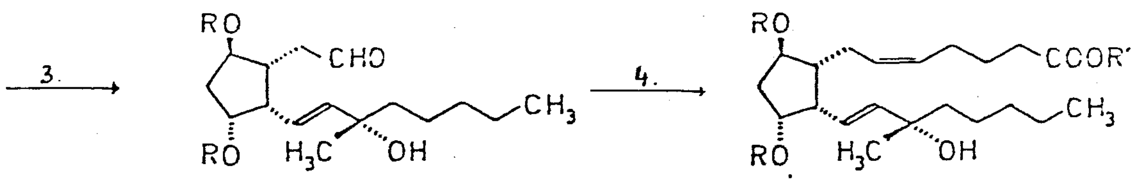


18

2,16-18: R = p-Phenylbenzoyl- (pPB)

| | R | R' |
|---|---|---|
| 19 | pPB | H |
| 20 | H | H |
| 21 | H | $CH_3$ |

1. Wittig-Horner-Reaktion (Alkylkette)
2. Grignard-Reaktion und Trennung der $C^{15}$-Epimeren*
3. Oxidation der primären OH-Gruppe an $C^{6}$
4. Wittig-Reaktion (Carboxylkette)

danach Abspaltung der Schutzgruppen und ggf. Veresterung

*Im Formelschema sind nur die 15-S-Epimeren wiedergegeben.

Bei der Synthese gemäß Beispiel 4 wird weitgehend auf die zusätzliche Einführung von Schutzgruppen verzichtet.

Nach der Reaktion von 2 mit dem Wittig-Horner-Reagens, vorzugsweise mit 2-Oxoheptylphosphonsäuredimethylester, wobei wie in Beispiel 2 nur die Verbindung der 12α-Reihe

entsteht, wird nicht aufgearbeitet, sondern bei tiefer Temperatur sofort eine Grignard-Verbindung, beispielsweise Methylmagnesiumiodid, hinzugegeben, unter Bildung des 15R/S-Diastereomerengemisches der Verbindungen 17 und (15R)-17.

Nach der Trennung der $C^{15}$-Epimeren wird je nach gewünschtem Endprodukt mit 17 oder mit (15R)-17 weitergearbeitet.

In der nächsten Stufe wird die $C^{6}$-Hydroxymethylgruppe zur Aldehydgruppe oxidiert, vorzugsweise mit Pyridiniumchlorochromat, unter Bildung der Aldehyde 18 bzw. (15R)-18.

Danach wird der jeweilige Aldehyd mit einem Wittig-Reagens, beispielsweise mit dem aus 4-Carboxybutyltriphenylphosphoniumbromid und Kalium-tert.-butylat gebildeten Ylid, zum mit den Schutzgruppen geschützten Prostaglandin 19 bzw. (15R)-19 umgesetzt.

Nach Abspaltung der Schutzgruppen erhält man in 8,4% Ausbeute das 15-Methyl-12-epi-$PGF_{2\beta}$ (20) und in 5,2% Ausbeute das (15R)-15-Methyl-12-epi-$PGF_{2\beta}$ ((15R)-20), jeweils bezogen auf 2.

Aus 20 bzw. (15R)-20 können beispielsweise durch Umsetzung mit etherischer Diazomethanlösung die Methylester 21 und (15R)-21 hergestellt werden.

Die erfindungsgemäßen 12-epi-Prostaglandine besitzen ein breites pharmakologisches Wirkungsspektrum, wobei ihre Wirkung auf den Kreislauf, insbesondere ihre vaskuläre Wirksamkeit, besonders ausgeprägt ist. Wie bei den natürlich vorkommenden Prostaglandinen zeigen die 15S- und 15R-Epimeren der erfindungsgemäßen 12-epi-Prostaglandine signifikante Unterschiede hinsichtlich ihrer pharmakologischen Wirkungsstärke und Wirkungsqualität.

Zum Beispiel wurde die kontrahierende Wirkung von (15R)-12-epi-$PGF_{2\beta}$ an einem isolierten Kaninchen-aortastreifen mit der des natürlichen $PGF_{2\alpha}$ verglichen. Die Dosis-Wirkungskurve des (15-R)-12-epi-$PGF_{2\beta}$ verläuft parallel zu der des $PGF_{2\alpha}$, jedoch mit einer ca. eine Zehnerpotenz niedrigeren Wirkungsstärke. Die Wirkung dieser erfindungsgemäßen Verbindung setzt bei etwa $3 \cdot 10^{-6}$ M/lein (vgl. Abb. 1). In dieser Konzentration ist das (15-S)-Epimere des 12 epi-$PGF_{2\beta}$ unwirksam.

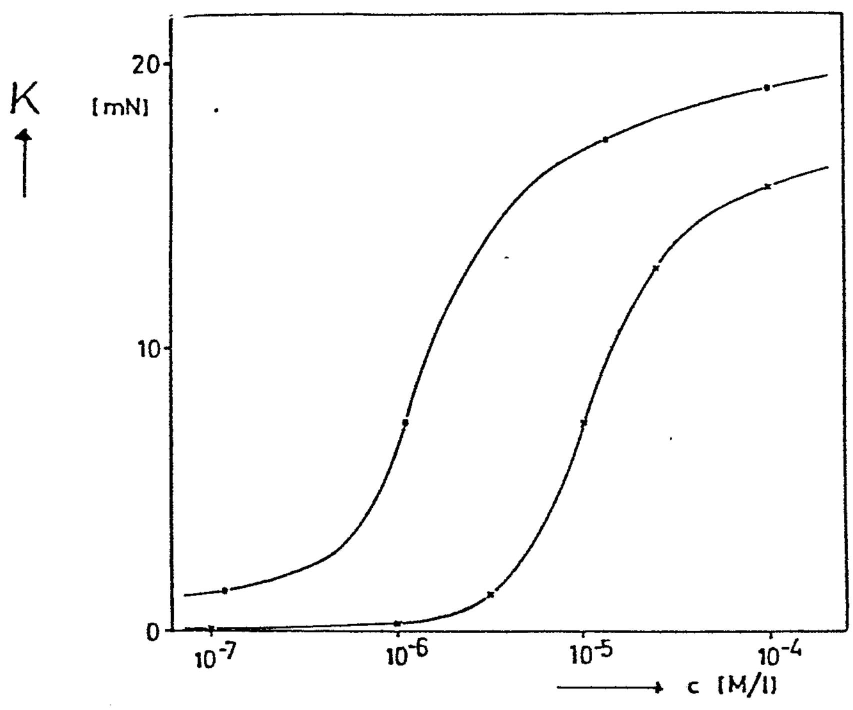


Abb. 1 Kontraktile Wirkung von (15R)-12-epi-$PGF_{2\beta}$ (x) und $PGF_{2\alpha}$ (.) auf isolierte Kaninchenaortastreifen in Abhängigkeit von der Dosis.
(c = Konzentration; K = Kontraktionskraft)
(Gemessen nach: J.R. Vane, Br.J. Pharmacol. Chemother. 23, 360-373 (1964)).

In mehreren anderen Tests wurden (15R)- und (15S)-12-epi-$PGF_{2\beta}$ sowie die beiden analogen erfindungsgemäßen 15-

Methylverbindungen mit dem bekannten $PGF_{2\alpha}$ verglichen. In allen Tests (Langendorff-Herz - gemessen: Koronarfluß, Herzfrequenz und Inotropie; isolierte Aorta - Tracheen- und Fundus-Streifen; Wirkung auf den Kreislauf von Meerschweinchen und Hund - gemessen: Blutdruck und Herzfrequenz) verlief die Wirkungsqualität des (15R)-12-epi-$PGF_{2\beta}$ parallel zu den Wirkungen des natürlichen $PGF_{2\alpha}$. (15S)-12-epi-$PGF_{2\beta}$ zeigte im Gegensatz dazu $PGE_2$-ähnliche Wirkung. Die 15-Methyl-Derivate der 12-epi-$PGF_{2\beta}$ zeigen analoge Unterschiede der Wirkungsqualität der jeweiligen 15R- und 15S-Epimeren.

Die erfindungsgemäßen Verbindungen werden vorzugsweise zur Herstellung pharmazeutischer Zubereitungen verwendet. Diese Zubereitungen enthalten mindestens eine der erfindungsgemäßen Verbindungen und können daneben noch übliche Träger- und Zusatzstoffe wie beispielsweise Wasser, pflanzliche Öle, Polyäthylenglykole, Glycerinester, Gelatine, Kohlenhydrate wie Lactose oder Stärke, Magnesiumstearat, Talk oder Vaseline, Konservierungsmittel, Stabilisierungsmittel, Gleitmittel, Netzmittel, Emulgatoren, physiologisch unbedenkliche Salze, Puffersubstanzen, Farbstoffe, Geschmacksstoffe und/oder Aromastoffe enthalten. Die Auswahl der Träger- und Zusatzstoffe hängt davon ab, ob die erfindungsgemäßen Verbindungen enteral, parenteral oder topikal appliziert werden sollen. Die Zubereitungen können als Arzneimittel sowohl in der Humanmedizin als auch in der Veterinärmedizin verwendet werden, insbesondere als Herz-Kreislaufmittel, und auch in Kombination mit anderen Wirkstoffen, beispielsweise Vitaminen oder bekannten, im Handel befindlichen Herz-Kreislauf-Mitteln.

Die Erfindung wird anhand der folgenden Beispiele weiter erläutert:

Die in den folgenden Beispielen verwendeten Substanznummern verweisen auf die Formelschemata im vorangehenden Teil.

Bei der Durchführung der Beispiele wurden folgende allgemeine Verfahren und Untersuchungsmethoden amgewandt:

Schmelzpunkte: Bestimmung in Kapillaren, nicht korrigiert. Spezif. Drehwerte: Polarimeter 141 (Perkin Elmer), "Uvasol"-Lösungsmittel (Merck). - IR-Spektren: Perkin Elmer 221. - Massenspektren: Vacuum Generators ZAB-2F (70 eV). - NMR-Spektren: Bruker WH-300; Varian CFT-20; TMS als interner Standard. - $R_F$-Werte: DC-Fertigplatten Kieselgel 60 $F_{254}$ (Merck); Sprühreagens: konz. Schwefelsäure/Formalin (9:1); Entwicklung ca. 10 min bei 120°C. - Säulenchromatographie: Glasdrucksäulen (Reichelt Chemietechnik); Membranpumpen "Prominent" (Chemie und Filter GmbH); Kieselsäure Mallinckrodt unter 100 mesh (Roth); die zu chromatographierende Substanz wird im Elutionsmittel gelöst, mit der 2- bis 4-fachen Menge Celite (Roth) versetzt und bis zur Trockne i. Vak. eingedampft. Der Rückstand wird auf die Drucksäule gegeben; Substanzmenge/verwendete Säulengröße: bis 1 g/80x1 cm, bis 4 g/100x2cm, bis 15 g/120x3 cm; Detektion erfolgt dünnschichtchromatographisch.

Beispiel 1 a:
Synthese der Ausgangsverbindung **2** ($R^1 = R^2$ = Benzyl)

(1S,6R,7R,9R)-(-)-2-Benzylthio-7,9-dibenzyloxy-3-oxabicyclo[4.3.0]nonan: Zu einer Suspension von 7.5 g (250 mmol) Natriumhydrid in Weißöl (80 % NaH) in 300 ml Dimethylformamid (DMF wird über eine Vigreux-Kolonne i.Vak. destilliert und 24 h über Molekularsieb 4 Å getrocknet) werden unter Stickstoffatmosphäre bei -10°C bis -5°C 11.3 g (1S,6R,7R,9R)-(-)-2-Benzylthio-7,9-dihydroxy-3-oxabicyclo[4.3.0]nonan in 150 ml Dimethylformamid innerhalb von 2 h getropft. Man rührt 1/2 h bei -5°C, tropft innerhalb von 1/2 h 40 ml Benzylbromid zu und läßt die Reaktionslösung auf Raumtemp. erwärmen. Das Ende der Reaktion wir 3 h nach Benzylbromid-Zugabe dünnschichtchromatographisch überprüft. Hat das Edukt abreagiert, wird erneut auf 0°C gekühlt und 150 ml wasserfreies Methanol zugetropft. Nach 1 h Rühren werden 300 ml Wasser zugegeben. Dabei fällt ein weißer Niederschlag aus, der nach dem Absaugen aus ca. 600 ml n-Hexan/Cyclohexan (8:2) umkristallisiert wird. Farblose Kristalle, Ausb. 15.5 g (84 %), Schmp. 89-90°C (n-Hexan/Cyclohexan 8:2), $\underline{R}_F$ = 0.41 (n-Hexan/Ethylacetat 8:2), $[\alpha]_{589}^{20}$ = -222° (c=1 g in 100 ml $CHCl_3$). - $^1$H-NMR (300 MHz, $CDCl_3$) $\delta$ =7.40-7.19 (15H,Aromaten-H), 5.38 (s; 1H, 2-H), 4.56 - 4.37 (4H, $OC\underline{H}_2C_6H_5$), 4.09 ($m_c$; 1H, 4-$H_A$), 3.97 ($m_c$;1H, 9-H), 3.82 (d; 1H, $SC\underline{H}_2C_6H_5$), 3.80 ($m_c$; 1H, 7-H), 3.67 ($m_c$; 1H, 4-$H_B$), 3.66 (d; 1H, $SC\underline{H}_2C_6H_5$), 2.42 ($m_c$; 1H, 6-H), 2.24 ($m_c$; 1H, 1-H), 2.15 ($m_c$, $^2\underline{J}$ = 14.7 Hz; 1H, 8-$H_A$), 1.96 ($m_c$, $^2\underline{J}$ = 14.7 Hz; 1H, 8-$H_B$), 1.74-1.56 (m; 2H, 5-H). - $^{13}$C-NMR (75.46 MHz, $CDCl_3$): $\delta$ = 138.92-126.78 (C-Aromaten), 84.36 (d; C-9), 80.60 (d; C-7), 77.96 (d; C-2), 71.52/71.01 (t; $O\underline{C}H_2C_6H_5$), 58.55 (t; C-4), 44.04 (d; C-6), 39.71 (d; C-1), 38.41 (t; C-8), 34.42 (t; $S\underline{C}H_2C_6H_5$), 27.11 (t; C-5).

| | | | | |
|---|---|---|---|---|
| $C_{29}H_{32}O_3S$ (460.5) | Ber. | C 75.65 | H 6.96 | S 6.96 |
| | Gef. | C 75.55 | H 7.15 | S 6.68 |

<u>(1S,6R,7R,9R)-7,9-Dibenzyloxy-2-hydroxy-3-oxabicyclo[4.3.0]nonan (2) ($R^1$ = $R^2$ = Benzyl):</u>

Zu einer Lösung von 6.3 g (19.7 mmol) Quecksilber(II)acetat in 170 ml Wasser werden 4.2 g (19.4 mmol) Quecksilber(II)oxid gegeben und innerhalb von 20 min 15.5 g (33.7 mmol) (1S,6R,7R,9R)-2-benzylthio-7,9-dibenzyloxy-3-oxabicyclo[4.3.0]nonan in 500 ml Aceton zugetropft. Man rührt 1 h. Der ausgefallene Niederschlag wird abfiltriert und zweimal mit 100 ml Wasser/Aceton (1:1) gewaschen. Die vereinigten Filtrate werden i.Vak. auf ca. 250 ml Lösung eingeengt und einmal mit 400 ml und zweimal mit

200 ml Ethylacetat ausgeschüttelt. Die Ethylacetatphasen werden 2 h mit 40 g wasserfreiem Kaliumcarbonat gerührt. Die organische Phase wird abdekantiert, über Nacht mit wasserfreiem Natriumsulfat getrocknet und i.Vak. zur Trockne eingedampft. Der Rückstand wird säulenchromatographisch gereinigt. Die Elution wird nacheinander mit 1 l Toluol/Aceton (95:5)und 1 l Toluol/Aceton (9:1) ausgeführt. Farbloses Oel. Ausb. 10.8 g (90 %), $R_F$ = 0.37 (Benzol/Aceton 8:2). Da 2 in Lösung als $C_2$-Epimerengemisch vorliegt, werden das $^1H$- und $^{13}C$-NMR-Spektrum nicht beschrieben.

$C_{22}H_{26}O_4$ (354.4) Ber. C 74.58 H 7.34
Gef. C 74.79 H 7.52

Beispiel 1 b:
Synthese der Ausgangsverbindung 2 ($R^1 = R^2$ = p-Phenylbenzoyl)

(1S,6R,7R,9R)-(-)-2-(Benzylthio)-7,9-bis(p-phenylbenzoyloxy)-3-oxabicyclo[4.3.0]nonan:
8.5 g (30.3 mmol) (1S,6R,7R,9R)-(-)-2-(Benzylthio)-3-oxabicyclo[4.3.0]nonan-7,9-diol (über $P_4O_{10}$ i.Vak. getrocknet) werden in 30 ml wasserfreiem Pyridin gelöst und bei 0°C mit 13.8 g (63.6 mmol) p-Phenylbenzoylchlorid versetzt. Man läßt 2 h bei Raumtemperatur rühren und gibt 100 g Eis zu der Reaktionslösung. Nachdem das Eis geschmolzen ist, wird das ausgefallene Produkt in 100 ml destilliertem Ethylacetat aufgenommen und die wäßrige Phase dreimal mit je 100 ml Ethylacetat ausgeschüttelt. Die vereinigten organischen Phasen werden mit 200 ml Wasser, 200 ml gesättigter $NaHCO_3$-Lösung und nochmals zweimal mit je 200 ml Wasser gewaschen. Das über wasserfreiem $Na_2SO_4$ getrocknete Ethylacetat wird mit wenig Celite im Rotationsverdampfer zur Trockne eingedampft und der Rückstand auf einer Säule (25 cm x 3 cm; Kieselgel 0.02 - 0.5 mm) mit 1.2 l Toluol als Elutionsmittel gereinigt. Gelbliches amorphes Produkt.

Ausb. 18.2 g (94 %), $\underline{R}_F$ = 0.75 (Toluol/Aceton 9:1), $[\alpha]^{20}_{589}$ = -206.8 ($\underline{c}$ = 0.9 g in 100 ml $CHCl_3$). - $^1\underline{H-NMR}$ (300 MHz, $CDCl_3$): $\delta$ = 8.18 - 7.33 (m; 18H, Aromaten-H), 7.30 - 7.15 (m; 5H, $-S-CH_2-C_6\underline{H}_5$), 5.67 ($m_c$; 1H, 9-H), 5.47(s;1H,2-H),5.29($m_c$;1H,7-H),4.21($m_c$;1H,4-H),3.80-3.72 (m; 1H, 4-H), AB-Signal ($\delta_A$ = 3.80 , $\delta_B$ = 3.66, $\underline{J}$ = 13.25 Hz; 2H, $-S-C\underline{H}_2-C_6H_5$), 2.68 - 2.42 (m; 4H, 6-H, 8-H und 1-H), 1.92 - 1.79 (m; 2H, 5-H). - $^{13}\underline{C-NMR}$ (75.46 MHz, $CDCl_3$): $\delta$ = 166.20 und 166.02 (s; $\underline{C}$=O), 145.98, 145.86, 140.20 und 140.05 (s; C-Aromaten), 138.31 (s; $-S-CH_2-\underline{C}_6H_5$), 130.37 - 126.93 (d; C-Aromaten) 79.69 (d; C-2), 78.01 (d; C-9), 76.24 (d; C-7), 58.09 (t; C-4), 43.83 (d; C-1), 40.77 (d; C-6), 39.54 (t; C-8), 34.51 (t; $-S-\underline{C}H_2-C_6H_5$), 25.91 (t; C-5).

| | | | | |
|---|---|---|---|---|
| $C_{41}H_{36}O_5S$ (640.3) | Ber. | C 76.85 | H 5.66 | S 5.00 |
| | Gef. | C 77.05 | H 5.90 | S 4.95 |

<u>(1S,6R,7R,9R)-(+)-7,9-Bis(p-phenylbenzoyloxy)-3-oxabicyclo[4.3.0]nonan-2-ol (2, $R^1 = R^2$ = p-Phenylbenzoyl):</u>

18.2 g (28.4 mmol) (1S,6R,7R,9R)-(-)-2-(Benzylthio)-7,9-bis(p-phenylbenzoyloxy)-3-oxabicyclo[4.3.0]nonan werden in 500 ml Aceton gelöst. Unter Rühren fügt man bei Raumtemp. 20 ml Wasser und 3.1 g (14.2 mmol) Quecksilber (II)-oxid hinzu und läßt eine Lösung von 5.0 g (15.5 mmol) Quecksilber(II)-acetat in 100 ml Wasser rasch zufließen. Man läßt bei 35°C rühren und verdünnt mit 1 l Wasser. Die Reaktionslösung wird dreimal mit je 300 ml Dichlormethan extrahiert, die vereinigten organischen Phasen werden zweimal mit je 200 ml Wasser gewaschen, über wasserfreiem $Na_2SO_4$ getrocknet und i.Vak. zur Trockne eingedampft. Der Rückstand wird in 50 ml Dichlormethan aufgenommen und filtriert. Das Filtrat versetzt man mit 150 ml Toluol und läßt ca 12 h bei Raumtemp. stehen. Das auskristallisierte 2 wird abgesaugt und die Mutterlauge auf 50 ml eingedampft, wobei Quecksilbersalze auskristallisieren. Man saugt ab und dampft auf ca. 20 ml ein, wobei weiteres 2 auskristallisiert. 2 liegt in Lösung

in einem C-2-Epimerengemisch vor. Ausb. 11,2 g (74 %). Schmp. 161-163° C, $\underline{R}_F$ = 0.30 (Toluol/Aceton 9:1), $[\alpha]^{20}_{589}$ = +28.8 ($\underline{c}$ = 1.0 g in 100 ml $CHCl_3$). - $^{1}$<u>H-NMR</u> (300 MHz, $CDCl_3$): $\delta$ = 8.18 - 8.05, 7.70 - 7.58 und 7.50 - 7.35 (m; 18H, Aromaten-H), 5.78 ($m_c$; 1H, 9-H), 5.43 (s; 1H, 2-H), 5.38 ($m_c$; 1H, 7-H), 4.06 ($m_c$; 1H, 4-H), 3.73 ($m_c$; 1H, 4-H), 2.83 - 2.54 (m; 4H, 1-H, 6-H und 8-H), 1.92 - 1.80 (m; 2H, 5-H), 1.60 (s; 1H, O<u>H</u>). $^{13}$<u>C-NMR</u> (75.46 MHz, $CDCl_3$: Das in höherer Konzentration vorliegende C-2-Epimere wird an erster Stelle angegeben. $\delta$ = 166.26 (s; <u>C</u>=O), 145.95 und 140.14 (s; C-Aromaten), 130.79 - 127.20 (d; C-Aromaten), 91.22/94.13 (d; C-2), 79.12/78.97 (d; C-9), 75.22/76.63 (d; C-7), 58.45/63.45 (t; C-4), 44.25/46.37 (d; C-1), 40.26/43.32 (d; C-6), 39.36/39.69 (t; C-8), 25.22/25.79 (t; C-5).

| | | | |
|---|---|---|---|
| $C_{34}H_{30}O_6$ (534.6) | Ber. | C 76.39 | H 5.66 |
| | Gef. | C 76.36 | H 5.61 |

<u>Beispiel 1 c:</u>

<u>Synthese der Ausgangsverbindung $\underline{2}$ ($R^1$ = p-Nitrobenzoyl, $R^2$ beliebig, vgl. S. )</u>

<u>(1S,6R,7R)-(-)-2-(Benzylthio)-7-(p-nitrobenzoyloxy)-9-oxo-3-oxabicyclo[4.3.0]nonan:</u>

9.3 g (33.4 mmol) (1S,6R,7R)-(-)-2-Benzylthio-7-hydroxy-9-oxo-3-oxabicyclo[4.3.0]nonan ($\underline{1}$) werden in 60 ml Pyridin gelöst und bei -20° C mit 6.5 g (35 mmol) p-Nitrobenzoylchlorid versetzt. Es wird 1 h gerührt, wobei man langsam die Temp. auf 0° ansteigen läßt, dann wird noch 2 h bei Raumtemp. weitergerührt. Schließlich wird auf Eiswasser gegossen, abgesaugt und mit Eiswasser pyridinfrei gewaschen. Das Produkt wird in Aceton unter Erwärmen gelöst und durch Versetzen mit Isopropanol auskristallisiert. Nach nochmaligem Umkristallisieren schwach gelbliche Kristalle, Ausb. 10.3 g (72 %), Schmp. 148-150° C. $\underline{R}_F$ = 0.72 ($CH_2Cl_2$/Aceton 95:5); $\underline{R}_F$ = 0.65 (Benzol/Aceton 4 : 1)

$\underline{R_F}$ = 0.60 (Toluol/Aceton 4:1) (Kieselgel auf Folie F 1440, Schleicher u. Schüll)
$[\alpha]^{20}_{589}$ =-134 (c=1.3 g in 100 ml $CHCl_3$-Lösung).

$C_{22}H_{21}NO_6S$ (427.5) Ber. C 61.81 H 4.95 N 3.28 S 7.50
Gef. C 61.66 H 5.22 N 3.12 S 7.27

$\underline{^1H\text{-}NMR}$ (300 MHz, $CDCl_3$): $\delta$ =8.32-8.12 (m; 4H, p-Nitroaromaten-H), 7.36 - 7.23 (m; 5H, Aromaten-H), 5.67 (s; 1H, 2-H, 5.38 (d, $^3\underline{J}$ = 6.19 Hz; 1H, 7-H), 4.18 ($m_c$; 1H, 4-H), AB-Signal ($\delta_A$ = 3.83, $\delta_B$ = 3.72, $\underline{J}$ = 13.27 Hz; 2H, $\underline{CH_2}C_6H_5$), 3.59 (m; 1H, 4'-H), 2.95 (m; 1H, 6-H), ABX-Signal ($\delta_A$ = 2.82, $\delta_B$ = 2.54, $^2\underline{J}$ = 20.35, $^3\underline{J}_{AX}$ = 6.19, $^3\underline{J}_{BX}$ = 0 Hz; 2H, 8-H u. 8'-H), 2.62 (d, $^3\underline{J}$ = 7.08 Hz; 1H, 1-H), 1.91 (m; 1H, 5-H), 1.27 (m; 1H, 5'-H). $\underline{^{13}C\text{-}NMR}$ (75.46 MHz, $CDCl_3$): $\delta$= 210.21 (s; C-9), 164.08 (s; $-C_6H_4\underline{C}O$), 138.0 - 123.7 (C-Aromaten), 77.02 (d; C-2), 75.16 (d; C-7), 56.89 (t; C-4), 49.97 (d; C-1), 41.13 (t: C-8), 37.60 (d; C-6), 34.81 (t; $\underline{CH_2}C_6H_5$), 25.55 (t; C-5).

<u>(1S,6R,7R,9R)-(-)-2-(Benzylthio)-7-(p-nitrobenzoyloxy)-9-hydroxy-3-oxabicyclo[4.3.0]nonan</u> und

<u>(1S,6R,7R,9S)-(-)-2-(Benzylthio)-7-(p-nitrobenzoyloxy)-9-hydroxy-3-oxabicyclo[4.3.0]nonan</u>:

9.9 g (23.2 mmol) (1S,6R,7R)-(-)-2-Benzylthio-7-(p-nitrobenzoyloxy)-9-oxo-3-oxabicyclo[4.3.0]nonan werden in 200 ml THF gelöst und zu einer Suspension von 0.4 g $NaBH_4$ (10.6 mmol) in 300 ml THF und 6 ml $H_2O$ bei -20° zugetropft. Danach wird noch 1 h bei Raumtemp. weitergerührt. Schließlich werden ca. 200 ml $H_2O$ zugegeben und dann eine Lösung von $NaH_2PO_4$ zur Zersetzung überschüssigen Natriumboranats. Nach Zugabe von Ether wird die wäßrige Phase abgetrennt und mehrmals mit Ether ausgeschüttelt. Die organ. Phase wird über Natriumsulfat getrocknet und eingedampft. Das Reaktionsgemisch wird nun in Dichlormethan gelöst, auf Celite aufgebracht und über eine Drucksäule mit Dichlormethan/Aceton (99:1) aufgetrennt. Die Trennung wurde dünnschichtchromatographisch auf Kieselgel mit $CH_2Cl_2$/Aceton (95:5) verfolgt. Ausbeute 5.0 g (9R)-2 (50.5 %) Krist. aus Ether/Petrolether (60-70°C) und 2.6 g (9S)-2 (26.2 %) Krist. aus Tetrachlormethan.

(9R)-2 : Schmp. 92-93°C $R_F$ = o.40 (Toluol/Aceton 4:1)
bzw. $R_F$ = 0.40 (Dichlormethan/Aceton 95:5)
(Kieselgel auf Folie F 1440, Schleicher u.Schüll)

$[\alpha]^{20}_{589}$ = -229 (0.02088 g in 2 ml $CHCl_3$-Lösung)

| | | | | | |
|---|---|---|---|---|---|
| $C_{22}H_{23}NO_6S$ (429.48) | Ber. | C 61.53 | H 5.40 | N 3.26 | S 7.46 |
| | Gef. | C 61.70 | H 5.52 | N 3.31 | S 7.36 |

$^1$H-NMR (300 MHz, $CDCl_3$): $\delta$ = 8.3 - 8.1 (m; 4H, p-Nitroaromaten), 7.4 - 7.2 (m; 5H, Aromaten), 5.42 (s; 1H, 2-H), 5.26 ($m_c$, J = 2 Hz, J = 6.6 Hz; 4.47 (m; 1H, 9-H), 4.26 ($m_c$, $^2J$ = 6.6Hz; 1H, 4-H), AB-Signal ($\delta_A$ = 3.81, $\delta_B$ = 3.70, J = 13.5Hz; 2H, $CH_2C_6H_5$), ~ 3.7 (m; 1H, 4'-H), 2.88 ($s_{br.}$; 1H, 9-OH), ~ 2.5 (m; 1H, 6-H), 2.43 ($m_c$, $^2J$ = 15.9 Hz; 1H, 8-H), 2.24 ($m_c$; 1H, 1-H), 2.19 ($m_c$, $^2J$ = 15.9 Hz; 1H, 8'-H), 1.9 - 1.7 (m; 2H, 5-H u.5'-H).

$^{13}$C-NMR (75.46 MHz, $CDCl_3$): = 164.3 (s; $-C_6H_4CO$), 150 - 123 (Aromaten-C), 81.42 (d; C-2), 78.58 (d; C-7), 74.68 (d; C-9), 58.09 (t; C-4), 44.31 (d; C-1), 41.85 (t; C-8), 39.90 (d; C-6), 34.21 (t; S-$CH_2$-), 26.27 (t; C-5).

(9S)-2: Schmp. 108-111°C $R_F$ = 0.37 (Toluol/Aceton 4:1)
bzw. $R_F$ = 0.27 (Dichlormethan/Aceton 95:5)
(Kieselgel auf Folie F 1440, Schleicher u. Schüll)

$[\alpha]^{20}_{589}$ = -176 (0.01972 g in 2 ml $CHCl_3$-Lösung).

| | | | | | |
|---|---|---|---|---|---|
| $C_{22}H_{23}NO_6S$ (429.48) | Ber. | C 61.53 | H 5.40 | N 3.26 | S 7.46 |
| | Gef. | C 61.26 | H 5.19 | N 3.06 | S 7.45 |

$^1$H-NMR (300 MHz, $CDCl_3$): $\delta$ = 8.32 - 8.12 (m; 4H, p-Nitroaromaten), 7.4 - 7.2 (m; 5H, Aromaten), 5.18 (s; 1H, 2-H), 5.0 (d; 1H, 7-H), 4.47 ($m_c$; 1H, 9-H), 4.17 ($m_c$; 1H, 4-H), AB-Signal ($\delta_A$ = 3.83, $\delta_B$ = 3.72, $J_{AB}$ = 13.5 Hz; 2H, $-CH_2C_6H_5$), 3.59 ($m_c$; 1H, 4'-H), 2.77 ($m_c$; 1H, 8-H), 2.54 ($m_c$; 1H, 6-H), 2.18 ($m_c$; 1H, 1-H), 1.9 ($s_{br.}$; 1H, 9-OH), 1.86-1.66 (m; 2H, 5-H u. 8'-H), 1.35 ($m_c$; 5-H).

$^{13}$C-NMR (75.46 MHz, $CDCl_3$): $\delta$ = 164.2 (s; $-C_6H_4CO$), 150.5-123.4 (Aromaten-C), 79.46 (d; C-2), 78.92 (d; C-7), 72.78 (d; C-9), 58.01 (t; C-4), 49.20 (d; C-1), 39.58 (t; C-8), 39.13 (d; C-6), 34.37 (t; $-CH_2$-S), 24.76 (t; C-5).

Nach Einführung einer von $R^1$ = p-Nitrobenzoyl verschiedenen Schutzgruppen (vgl. S. ) wird die Synthese der Prostaglandine analog zu den Beispielen 2 - 4 durchgeführt.

Beispiel 2:

Synthese des 12-epi-$PGF_{2\beta}$ aus 2 ($R^1$ = $R^2$ = Benzyl)

2-Decarboxy-15-dehydro-9,11-di-O-benzyl-6-hydroxy-2,3,4,5-tetranor-12-epi-$PGF_{1\beta}$ (3):

Zu einer Suspension von 420 mg (14 mmol) Natriumhydrid (80 % in Weißöl) in 200 ml wasserfreiem Dimethoxyethan werden bei Raumtemperatur in einer mehrmals ausgeflammten Apparatur unter $N_2$-Atmosphäre 3.7 ml (17.8 mmol) 2-Oxoheptylphosphonsäuredimethylester gegeben. Man läßt 1 h rühren und tropft 4.1 g (11.5 mmol) 2 ($R^1$ = $R^2$ = Benzyl) in 20 ml Dimethoxyethan zu. Die Reaktionslösung wird 4 h bei 40°C Badtemp. kräftig gerührt. Durch Zugabe von 1.6 ml Eisessig wird überschüssiges Phosphonat zerstört. Die Lösung wird mit 160 ml Wasser verdünnt und dreimal mit je 120 ml Ethylacetat extrahiert. Die vereinigten organischen Phasen werden zweimal mit je 100 ml 10proz. Natriumchloridlösung ausgeschüttelt. mit wasserfreiem Natriumsulfat getrocknet und i.Vak. zur Trockne eingedampft. Der Rückstand wird säulenchromatographisch getrennt. Elution mit Toluol/Aceton (96:4).

 0144459

Fraktion 1: (1S,6R,7R,9R)-7,9-Dibenzyloxy-2-(2'-oxo-heptyl)-3-oxabicyclo[4.3.0]nonan als farbloses Oel. $R_F$ 0.63 (Toluol/Aceton 8:2), Ausb. 12 %.

Fraktion 2: 3 als farbloses Oel. Ausb. 4.3 g (83 %), $R_F$ = 0.35 (Toluol/Aceton 8:2). - IR (Film): 3450 (OH), 1670 (C=O), 1630 (C=C), 985 $cm^{-1}$ (C=C, E-ständig). $^1$H-NMR (300 MHz, $CDCl_3$, H/D-Austausch): $\delta$ = 7.40 - 7.23 (10H, Aromaten-H), 6,74 (dd; $J_{12/13}$ = 10.3 Hz, $J_{13/14}$ = 16.2 Hz; 1H, 13-H), 6.16 (d; $J_{13/14}$ = 16.2 Hz; 1H, 14-H), 4.56-4.39 (4H, $OCH_2C_6H_5$), 4.21 ($m_c$; 1H, 11-H), 3.96 ($m_c$; 1H, 9-H), 3.60 ($m_c$; $J$ = 5.9 Hz; 2H, 6-H), 3.08 ($m_c$; $J_{12/13}$ = 10.3 Hz; 1H, 12-H), 2.53 (t; $J$ = 7.5 Hz; 2H, 16-H), 2.32 - 2.13 (m; 3H, 8-H, 10-H), 1.78 - 1.46 (m; 4H, 7-H, 17-H), 1.40 - 1.20 (m; 4H, 18-H, 19-H), 0.88 (t; 3H, 20-H). - $^{13}$C-NMR (75.46 MHz, $CDCl_3$, Tab. 4).

| | | | |
|---|---|---|---|
| $C_{29}H_{38}O_4$ (450.6) | Ber. | C 77.30 | H 8.50 |
| | Gef. | C 77.38 | H 8.25 |

2-Decarboxy-15-dehydro-9,11-di-O-benzyl-6-(p-phenyl-benzoyloxy)-2,3,4,5-tetranor-12-epi-$PGF_{1\beta}$ (4):
4.3 g (9.55 mmol) 3 werden in 5 ml wasserfreiem Pyridin gelöst, mit 2.3 g (10.5 mmol) p-Phenylbenzoylchlorid versetzt und 1 h bei 0°C sowie 1 h bei Raumtemp. gerührt. Man gibt 100 ml Cyclohexan zu und extrahiert dreimal mit je 50 ml Wasser. Die Cyclohexanphase wird mit wasserfreiem Natriumsulfat getrocknet und das Lösungsmittel i.Vak. entfernt. Der Rückstand wird zur Analyse säulenchromatographisch mit Petrolether (60-70°C)/Aceton 95:5) als Elutionsmittel gereinigt. Zur weiteren Synthese kann das Rohprodukt verwendet werden. Farbloses Oel. Ausb. 5.6 g (93 %), $R_F$ = 0.39 (Toluol/Aceton 95:5). - IR (Film): 1720 (C=O), 1675 (C=O), 1630 (C=C), 985 $cm^{-1}$ (C=C, E-ständig). - $^1$H-NMR (300 MHz, $CDCl_3$): $\delta$ = 8.09-7.22 (19H, Aromaten-H), 6.84 (dd; $J_{12/13}$ = 10.6 Hz; $J_{13/14}$ = 15.5 Hz; 1H, 13-H), 6.23 (d; $J_{13/14}$ = 15.5 Hz; 1H, 14-H), 4.55 - 4.43 (4H, $OCH_2C_6H_5$), 4.36 ($m_c$; 2H, 6-H),

4.21 ($m_c$; 1H, 11-H), 3.97 ($m_c$; 1H, 9-H), 3.16 ($m_c$; 1H, 12-H), 2.52 (t; J = 7.5 Hz; 2H, 16-H), 2.33 - 2.21 (m; 2H, 8-H, 10-$H_A$), 2.16 ($m_c$; 1H, 10-$H_B$), 1.89 ($m_c$; 2H, 7-H), 1.60 ($m_c$; 2H, 17-H), 1.28 ($m_c$; 4H, 18-H, 19-H), 0.88 (t; 3H, 20-H). - $^{13}$C-NMR (300 MHz, $CDCl_3$, Tab.4).

$C_{42}H_{46}O_5$ (630.8) Ber. C 79.90 H 7.40
Gef. C 79.79 H 7.42

2-Decarboxy-9,11-di-O-benzyl-6-(p-phenylbenzoyloxy)-2,3,4,5-tetranor-12-epi-$PGF_{1\beta}$ (5) und sein 15R-Diastereomeres (15R)-5:

Zu einer Suspension von 7.35 g (54 mmol) Zinkchlorid in 11 ml wasserfreiem Dimethoxyethan gibt man bei -5°C portionsweise 1.75 g (41 mmol) Natriumborhydrid und rührt über Nacht unter Erwärmenlassen auf Raumtemp.. Anschliessend werden bei -20°C 5.7 g (9 mmol) 4 in 15 ml Dimethoxyethan zugetropft. Man rührt jeweils 1 1/2 h bei -20°C, 1 h bei 0°C und 3 h bei Raumtemp., zersetzt bei 0°C mit 15 ml Wasser und fügt 500 ml Ethylacetat zu. Der Niederschlag wird abgesaugt und zweimal mit je 50 ml Ethylacetat gewaschen. Die vereinigten organischen Phasen werden zweimal mit je 250 ml 10proz. Natriumchloridlösung ausgeschüttelt, mit wasserfreiem Natriumsulfat getrocknet und i.Vak. eingedampft. Die Diastereomeren 5 und (15R)-5 werden säulenchromatographisch getrennt. Elution mit Toluol/Aceton (96:4).

Fraktion 1: 5 als farbloses Oel. Ausb. 2.15 g (38 %), $R_F$ = 0.62 (Toluol/Acetat 8:2). - IR (Film): 3500 (OH), 1720 (C=O), 970 $cm^{-1}$ (C=C, E-ständig). - $^1$H-NMR (300 MHz, $CDCl_3$, H/D-Austausch): $\delta$ = 8.11 - 7.24 (19H, Aromaten-H), 5.69 ($m_c$; $J_{13/14}$ = 15.3 Hz; 1H, 13-H oder 14-H), 5.59 ($m_c$; $J_{13/14}$ = 15.3 Hz; 1H, 13-H oder 14-H), 4.57 - 4.38 (4H, $OCH_2C_6H_5$), 4.51 ($m_c$; 1H, 6-$H_A$), 4.25 - 4.11 (m̃; 3H, 6-$H_B$, 11-H, 15-H), 3.85 ($m_c$; 1H, 9-H), 3.03 ($m_c$; 1H, 12-H), 2.13 ($m_c$; 3H, 8-H, 10-H), 1.94 ($m_c$; 2H, 7-H), 1.55 ($m_c$; 2H, 16-H), 1.42 - 1.20 (m; 6H, 17-H, 18-H, 19-H),

0.86 (t; 3H, 20-H). - $^{13}$C-NMR (75.46 MHz, $CDCl_3$, Tab.4).

$C_{42}H_{48}O_5$ (632.8) Ber. C 79.71 H 7.65
Gef. C 79.86 H 7.76

Fraktion 2: (15R)-5 als farbloses Oel. Ausb. 3 g (52 %), $R_F$ = 0.57 (Toluol/Aceton 8:2), - IR (Film): 3450 (OH), 1720 (C=O), 970 $cm^{-1}$ (C=C, E-ständig). - $^1$H-NMR (300 MHz, $CDCl_3$, H/D-Austausch); = 8.10 - 7.25 (19H, Aromaten-H), 5.59 ($m_c$; $J_{13/14}$ = 15.5 Hz; 2H, 13-H, 14-H), 4.56 - 4.41 (4H, $OCH_2C_6H_5$), 4.35 (t; J = 7.1 Hz; 2H, 6-H), 4.17 ($m_c$; 1H, 11-H), 4.05 (q; 1H, 15-H), 3.87 (q; 1H, 9-H), 3.03 (dt; 1H, 12-H), 2.16 ($m_c$; 3H, 8-H, 10-H), 1.90 ($m_c$; 2H, 7-H), 1.50 ($m_c$; 2H, 16-H), 1.36 - 1.20 (m; 6H, 17-H, 18-H, 19-H), 0.83 (t; 3H, 20-H). - $^{13}$C-NMR (20 MHz, $CDCl_3$, Tab.4).

$C_{42}H_{48}O_5$ (632.8) Ber. C 79.71 H 7.65
Gef. C 79.81 H 7.93

9,11-Di-O-benzyl-12-epi-PGF$_{2\beta}$ (10): 6-9 werden als Rohprodukte zur weiteren Reaktion eingesetzt.

6: 3 g (4.75 mmol) 5 werden in 17 ml Dichlormethan gelöst und mit 1.3 g (15.4 mmol) Dihydropyran sowie 0.5 ml einer 2proz. Lösung von p-Toluolsulfonsäure in Tetrahydrofuran versetzt. Es wird 3 h gerührt und anschließend mit 1.1 ml Pyridin neutralisiert. Die Reaktionslösung wird i.Vak. eingedampft und gleich zu 7 umgesetzt.

7: 6 wird in 30 ml Methanol mit 690 mg (5 mmol) Kaliumcarbonat 25 h bei 35°C gerührt. Anschließend werden 150 ml Toluol zugegeben und zweimal mit Wasser extrahiert. Die organische Phase wird i. Vak. zur Trockne eingedampft und säulenchromatographisch gereinigt. Elution mit Toluol/Aceton (95:5). Ausb. 2.18 g (86 % bez. auf 5).

8: 1.72 g (8 mmol) Pyridiniumchlorochromat werden mit 8 g Aluminiumoxid (neutral) in einer Reibschale gut vermischt

und mit 132 mg (1.6 mmol) wasserfreiem Natriumacetat in 16 ml wasserfreiem Dichlormethan suspendiert. Man rührt 2 h und gibt 2.18 g (4.06 mmol) 7 in 16 ml Dichlormethan zu. Nach 3 h Rühren ist die Reaktion beendet. Man verdünnt mit 32 ml wasserfreiem Diethylether und dekantiert von den Salzen ab. Der Rückstand wird noch achtmal mit je 30 ml Diethylether versetzt, aufgerührt und die Lösung jeweils dekantiert. Die vereinigten Etherlösungen werden über $Mg_2O_8Si_3 . 2H_2O$ abgesaugt. Man spült mit 100 ml Ether nach und dampft die Etherlösung i.Vak. zur Trockne ein. Ausb. 2 g (93 %).

9: Die Herstellung des Ylids sowie die Wittig-Reaktion werden unter Stickstoffatmosphäre und unter Feuchtigkeitsausschluß in einem mehrmals ausgefalmmten Reaktionsgefäß ausgerührt. Zu einer Suspension von 3.14 g (7 mmol) 5-Triphenylphosphonio-pentansäurebromid in 60 ml wasserfreiem Tetrahydrofuran werden bei Raumtemp. 1.51 g (13.5 mmol) Kalium-tert.butylat gegeben. Die Mischung wird 30 min gerührt. Zu der Lösung des Ylids werden bei -78°C 2 g (3.8 mmol) 8 in 40 ml wasserfreiem Tetrahydrofuran innerhalb von 10 min getropft. Es wird 5 min bei -78°C sowie 40 min bei 0°C gerührt. Man gibt soviel Citratpuffer (pH 4.5) zu, bis eine klare Lösung entsteht, verdünnt mit 120 ml Wasser und extrahiert dreimal mit je 70 ml Ethylacetat . Die vereinigten organischen Phasen werden i. Vak. eingedampft und gleich zu 10 umgesetzt.

10: 9 wird in 60 ml Eisessig/Wasser/Tetrahydrofuran (6:3:1) gelöst und 5 h bei 40°C gerührt. Danach wird die Reaktionslösung i.Vak. zur Trockne eingedampft und säulenchromatographisch gereinigt. Elution mit Toluol/Ethylacetat/Eisessig (90:9:1). Farbloses Oel. Ausb. 1.5 g (59 %), $R_F$ = 0.41 (Toluol/Ethylacetat/Methanol/Eisessig 80:20:5:1). - IR (Film): 3400 (OH), 1710 (C=O), 970 $cm^{-1}$ (C=C, E-ständig). - $^1H$-NMR (300 MHz, $CDCl_3$, H/D-Austausch): $\delta$ = 7.36 - 7.22 (10H, Aromaten-H), 5.67 ($m_c$;

$\underline{J}_{13/14}$ = 15.3 Hz; 1H, 13-H oder 14-H), 5.55 ($m_c$; $\underline{J}_{13/14}$ = 15.3 Hz; 2H, 5-H oder 6-H, 13-H oder 14-H), 5.38 ($m_c$; 1H, 5-H oder 6-H), 4.50 - 4.38 (4H, $OC\underline{H_2}C_6H_5$), 4.12 ($m_c$; 2H, 11-H, 15-H), 3.79 ($m_c$; 1H, 9-H), 2.96 ($m_c$; 1H, 12-H), 2.30 (t; $\underline{J}$ = 6.5 Hz; 2H, 2-H), 2.28 - 2.01 ($m_c$; 7H, 4-H, 7-H, 8-H, 10-H), 1.64 ($m_c$; 2H, 3-H), 1.50 ($m_c$; 2H, 16-H), 1.28 ($m_c$; 6H, 17-H, 18-H 19-H), 0.86 (t; 3H, 20-H). - $\underline{^{13}C\text{-NMR}}$ (75.46 MHz, $CDCl_3$, Tab. 4).

| | | | | |
|---|---|---|---|---|
| $C_{34}H_{46}O_5$ | (534.7) | Ber. | C 76.33 | H 8.67 |
| | | Gef. | C 76.15 | H 8.73 |

$\underline{12\text{-epi-PGF}_{2\beta}}$ (11): 1.5 g (2.8 mmol) 10 in 30 ml wasserfreiem Diethylether werden zu einer Lösung von 700 mg Natrium in 100 ml Diethylether / 120 ml Ammoniak innerhalb von 45 min getropft und 1 h gerührt. Zur Neutralisation werden 2.1 g Ammoniumchlorid zugegeben und das Ammoniak abgedampft. Man verdünnt mit 200 ml Ethanol, filtriert die anorganischen Salze ab und dampft die Reaktionslösung i. Vak. zur Trockne ein. Der Rückstand wird säulenchromatographisch mit Toluol/Ethylacetat/Methanol/Eisessig (30:64:4:2) als Elutionsmittel gereinigt. Farbloses Oel. Ausb. 0.75 g (82 %), $\underline{R}_F$ = 0.31 (Ethylacetat Isopropanol/Eisessig 80:10:2). - $\underline{^1H\text{-NMR}}$ (300 MHz, $CDCl_3$/$CD_3OD$ 9:1): $\delta$ = 5.65 ($m_c$; $\underline{J}_{13/14}$ = 15.3 Hz; 1H, 13-H oder 14-H), 5.57 ($m_c$; $\underline{J}_{13/14}$ = 15.3 Hz; 1H, 13-H oder 14-H), 5.50 ($m_c$; 2H, 5-H, 6-H), 4.36 (q; 1H, 11-H), 4.15 ($m_c$; 2H, 9-H, 15-H), 2.86 ($m_c$; 1H, 12-H), 2.31 (t; 2H, 2-H), 2.30 - 1.86 (m; 7H, 4-H, 7-H, 8-H, 10-H), 1.65 (quint; 2H, 3-H), 1.53 ($m_c$; 2H, 16-H), 1.30 ($m_c$; 6H, 17-H, 18-H, 19-H), 0.89 (t; 3H, 20-H). - $\underline{^{13}C\text{-NMR}}$ (75.46 MHz, $CDCl_3$, Tab.1).

| | | | | |
|---|---|---|---|---|
| $C_{20}H_{34}O_5$ | (354.5) | Ber. | C 67.77 | H 9.67 |
| | | Gef. | C 67.03 | H 9.31 |

$\underline{12\text{-epi-PGF}_{2\beta}\text{-methylester}}$ (12): 11 wird mit einer etherischen Diazomethanlösung verestert. Farbloses Oel.

$R_F$ = 0.31 (Chloroform/methanol 9:1). - IR (Film): 3370 (OH), 1735 (C=O), 970 $cm^{-1}$ (C=C, E-ständig). - $^1H$-NMR (300 MHz, $CDCl_3$, H/D-Austausch): $\delta$ = 5.69 ($m_c$; $J_{13/14}$ = 15.5 Hz; 1H, 14-H), 5.54 ($m_c$; $J_{13/14}$ = 15.5 Hz; 1H, 13-H), 5.42 ($m_c$; $J_{5/6}$ = 11 Hz; 2H, 5-H, 6-H), 4.34 ($m_c$; 1H, 11-H), 4.11 ($m_c$; 2H, 9-H, 15-H), 3.68 (s; 3H, $COOCH_3$), 2.87 ($m_c$; 1H, 12-H), 2.33 (t; 2H, 2-H), 2.25 ($m_c$; 1H, 7-$H_A$), 2.11 - 1.97(m; 5H, 4-H, 7-$H_B$, 10-H), 1.84 ($m_c$; 1H, 8-H), 1.67 ($m_c$; 2H, 3-H), 1.51 ($m_c$; 2H, 16-H), 1.30 ($m_c$; 6H, 17-H, 18-H, 19-H), 0.88 (t; 3H, 20-H). - $^{13}C$-NMR (75.46 MHz, $CDCl_3$, Tab. 1).

| | | | |
|---|---|---|---|
| $C_{21}H_{36}O_5$ (368.5) | Ber. | C 68.44 | H 9.85 |
| | Gef. | C 67.36 | H 9.89 |

9,11,15-Tris(p-phenylbenzoyl)-12-epi-$PGF_{2\beta}$-methylester: **12** wird mit p-Phenylbenzoylchlorid/wasserfreiem Pyridin verestert. Farblose Kristalle aus Ethylacetat. Schmp. 161-162° C, $R_F$ = 0.58 (Toluol/Aceton 95:5), $[\alpha]^{20}_{589}$ =-16° (c = 1 g in 100 ml Chloroform). IR (KBr): 1735 (C=O), 1705 (C=O), 980 $cm^{-1}$ (C=C, E-ständig). - $^1H$-NMR (300 MHz, $CDCl_3$): $\delta$ = 8.13 - 7.35 (m; 27H, Aromaten-H), 5.88 ($m_c$; $J_{13/14}$ = 15.5 Hz; 1H, 13-H), 5.70 ($m_c$; $J_{13/14}$ = 15.5 Hz; 1H, 14-H), 5.58 ($m_c$; 1H, 11-H), 5.51 - 5.32 (m; 4H, 5-H, 6-H, 9-H, 15-H), 3.57 (s; 3H, $COOCH_3$), 3.36 ($m_c$; 1H, 12-H) 2.63 ($m_c$; 1H, 7-$H_A$ oder 10-$H_A$), 2.45 - 2.26 (m; 4H, 7-$H_B$/10-H oder 7-H/10-$H_B$, 8-H), 2.20 (t; 2H, 2-H), 2.02 ($m_c$; 2H, 4-H), 1.75 - 1.49 (m; 2H, 16-H), 1.60 ($m_c$; 2H, 3-H), 1.30 ($m_c$; 2H, 17-H), 1.16 ($m_c$; 4H, 18-H, 19-H), 0.77 ($m_c$; 3H, 20-H). - $^{13}C$-NMR (75.46 MHz, $CDCl_3$): $\delta$ = 173.8 (s; C-1), 166.1, 165.7, 165.6 (s; C=O), 145.8 - 127.0 (C-Aromaten, C-5, C-6, C-13), 133.4 (d; C-14), 77.6 (d; C-11), 75.6, 75.0 (2d; C-9, C-15), 51.3 (q; $COOCH_3$), 48.4, 48.2 (2d; C-8, C-12), 37.7 (t; C-10), 34.5 (t; C-16), 33.3 (t; C-2), 31.6 (t; C-18), 26.9, 26.8 (2t; C-4, C-7), 24.8, 24.7 (2t; C-3, C-17), 22.4 (t; C-19), 14.0 (q; C-20).

| | | | |
|---|---|---|---|
| $C_{60}H_{60}O_8$ (909.1) | Ber. | C 79.28 | H 6.65 |
| | Gef. | C 79.14 | H 6.68 |

Synthese des (15R)-12-epi-PGF$_{2\beta}$ ((15R)-11):

Die 15R-epimeren Verbindungen (15R)-6 bis (15R)-12 werden analog dem vorstehenden Syntheseweg für das 12-epi-PGF$_{2\beta}$ (11) aus (15R)-5 hergestellt.

(15R)-9,11-Dibenzyl-12-epi-PGF$_{2\beta}$ ((15R)-10):

Farbloses Oel. Ausb. 60 % bez. auf (15R)-5, $R_F$ = 0.37 (Toluol/Ethalacetat/Methanol/Eisessig 80:20:5:1). - IR (Film): 3400 (OH), 1710 (C=O) 970 $cm^{-1}$ (C=C), E-ständig). - $^1H$-NMR (300 MHz, $CDCl_3$, H/D-Austausch): $\delta$ = 7.37 - 7.22 (10H, Aromaten-H) 5.59 ($m_c$; 2H, 13-H, 14-H), 5.40 ($m_c$; 2H, 5-H), 6-H), 4.50 - 4.37 (m; 4H, $OCH_2C_6H_5$), 4.09 ($m_c$; 2H, 11-H, 15-H), 3.77 ($m_c$; 1H, 9-H), 2.96 ($m_c$; 1H, 12-H), 2.28 ($m_c$; 2H, 2-H), 2.22 - 1.98 (m; 7H, 4-H, 7-H, 8-H, 10-H), 1.64 ($m_c$; 2H, 3-H), 1.51 ($m_c$; 2H, 16-H), 1.28 ($m_c$; 6H, 17-H, 18-H, 19H), 0.87 (t; 3H, 20-H). - $^{13}C$-NMR (75.46 MHz, $CDCl_3$, Tab.4).

| | | | |
|---|---|---|---|
| $C_{34}H_{46}O_5$ (534.7) | Ber. | C 76.37 | H 8.67 |
| | | C 76.09 | H 8.86 |

(15R)-12-epi-PGF$_{2\beta}$ ((15R)-11):

Farblose Kristalle aus Ethylacetat. Ausb. 76 %, Schmp. 105 - 106°C, $R_F$ = 0.18 (Ethylacetat/Isopropanol/Eisessig 80:10:2), $[\alpha]_{589}^{20}$ = -56° (c = 1 g in 100 ml Methanol). - IR (KBr): 3460 (OH), 3270 (OH), 1715 (C=O), 980 $cm^{-1}$ (C=C, E-ständig). - $^1H$-NMR (300 MHz, $CDCl_3/CD_3OD$ 9:1): $\delta$= 5.53 ($m_c$; $J_{13/14}$ = 15 Hz; 1H, 14-H), 5.45 ($m_c$; $J_{13/14}$ = 15 Hz; 1H, 13-H), 5.45 - 5.33 (m; 2H, 5-H, 6-H), 4.34 ($m_c$;1H,11-H),4.05($m_c$;2H,9-H,15-H),2.82($m_c$;1H,12-H),2.30(t; J=7.1 Hz;2H,2-H), 2.29 ($m_c$; 1H, 7-$H_A$), 2.16 - 1.91 (m; 5H, 4-H, 10-H, 7-$H_B$), 1.83 ($m_c$; 1H, 8-H), 1.66 ($m_c$; 2H, 3-H), 1.59 - 1.41 (m; 2H, 16-H), 1.29 ($m_c$; 6H, 17-H, 18-H, 19-H), 0.88 (t; 3H, 20-H). - $^{13}C$-NMR (75.46 MHz, $CDCl_3/CD_3OD$ 9:1, Tab.2).

| | | | |
|---|---|---|---|
| $C_{20}H_{34}O_5$ (354.5) | Ber. | C 67.77 | H 9.67 |
| | Gef. | C 67.68 | H 9.77 |

(15R)-12-epi-PGF$_{2\beta}$-methylester ((15R)-12):
Kristalle aus Diisopropylether, Ausb. 95 %, Schmp. 81 - 82°, $R_F$ = 0.27 (Chloroform/Methanol 9:1). $[\alpha]^{20}_{589}$ = -54° (c = 1 g in 100 ml Methanol). - IR (KBr): 3240 (OH), 1740 (C=O), 960 $cm^{-1}$ (C=C, E-ständig). - $^1$H-NMR (300 MHz, $CDCl_3$, H/D-Austausch): δ = 5.63 ($m_c$; $J_{13/14}$ = 15.3 Hz; 1H, 14-H), 5.54 ($m_c$, $J_{13/14}$ = 15.3 Hz; 1H, 13-H), 5.50 - 5.47 (m; 2H, 5-H, 6-H), 4.37 (q; 1H, 11-H), 4.15 ($m_c$; 1H, 9-H), 4.11 ($m_c$; 1H, 15-H), 3.67 (s; 3H, $COOCH_3$), 2.87 ($m_c$; 1H, 12-H), 2.32 (t; J = 7.3 Hz; 2H, 2-H), 2.26 ($m_c$; 1H, 7-$H_A$), 2.15 - 2.00 (m; 5H, 4-H, 7-$H_B$, 10-H), 1.88 ($m_c$; 1H, 8-H), 1.68 (quint; J = 7.3 Hz; 2H, 3-H), 1.56 - 1.45 (m; 2H, 16-H), 1.30 ($m_c$; 6H, 17-H, 18-H, 19-H), 0.90 (t; 3H, 20-H). - $^{13}$C-NMR (75.46 MHz, $CDCl_3$, Tab.2). - MS (70 eV):m/z = 350 (1.7 %, M-$H_2O$, ber. 350.2455, gef. 350.3467), 332 (4.1 %, $C_{21}H_{32}O_3$), 314 (1.9 %, $C_{21}H_{30}O_2$), 278 (34.4 %, $C_{18}H_{30}O_2$), 191 (14.9 %, $C_{13}H_{19}O$), 165 (15 %, $C_{11}H_{17}O$), 137 (41.2 %, $C_{10}H_{17}$), 99 (42 %, $C_6H_{11}O$), 67 (100 %, $C_5H_7$).

| | | | |
|---|---|---|---|
| $C_{21}H_{36}O_5$ (368.5) | Ber. | C 68.44 | H 9.85 |
| | Gef. | C 68.31 | H 9.87 |

Beispiel 3:
Synthese des PGF$_{2\beta}$ aus 2 ($R^1$ = $R^2$ = Benzyl)

(1R,2R,3R,4R)-(+)-1,4-Dibenzyloxy-2-(2-hydroxyethyl)-3-hydroxymethyl-cyclopentan (13):
7.8 g (20 mmol) 2 ($R^1$ = $R^2$ = Benzyl) werden in 100 ml wasserfreiem Tetrahydrofuran gelöst und innerhalb von 1/2 h bei 0°C zu einer Suspension von 1.5 g (39.5 mmol) $LiAlH_4$ in 300 ml Tetrahydrofuran getropft. Man läßt die Lösung unter Rühren innerhalb von 2 h auf Raumtemp. erwärmen. Zur Zerstörung von überschüssigem $LiAlH_4$ werden 50 ml Ethylacetat und 300 ml Wasser zugegeben. Es wird mit $CO_2$ neutralisiert, abgesaugt und die Salze mit Wasser gewaschen. Die wässrige Lösung wird viermal mit Ethylacetat extrahiert. Die organischen Phasen werden mit wasserfreiem Natriumsulfat getrocknet und i.Vak. zur Trockne

eingedampft. Der Rückstand wird aus 80 ml Toluol umkristallisiert. Farblose Kristalle. Schmp. 101 - 102°C (Toluol), Ausb. 7 g (90 %), $\underline{R_F}$ = 0.5 (Chloroform/Methanol 9:1), $[\alpha]^{20}_{589}$ = +83° (c = 1 g in 100 ml Chloroform). - $\underline{^1H\text{-NMR}}$ (300 MHz, $CDCl_3$): $\delta$ = 7.33 - 7.20 (10H, Aromaten-H), 4.61 - 4.39 (4H, $OC\underline{H}_2C_6H_5$), 4.30 ($m_c$; 1H, 4-H), 3.92 ($m_c$; 1H, 1-H), 3.75 ($m_c$; 2H, $C\underline{H}_2OH$), 3.65 ($m_c$; 2H, $CH_2C\underline{H}_2OH$), 2.47 ($m_c$; 1H, 3-H), 2.20 - 1.98 (m; 3H, 2-H, 5-H), 1.75 ($m_c$; 1H, $C\underline{H}_2CH_2OH$), 1.61 ($m_c$; 1H, $C\underline{H}_2CH_2OH$), - $\underline{^{13}C\text{-NMR}}$ (75.46 MHz, $CDCl_3$): $\delta$ = 138.0 - 127.56 (C-Aromaten), 82.95 (d; C-4), 80.56 (d; C-1), 71.94 (t; $O\underline{C}H_2C_6H_5$), 71.90 (t; $O\underline{C}H_2O_6H_5$), 61.73 (t; $CH_2OH$), 59.90 (t; $CH_2$-$\underline{C}H_2$-OH), 46.24 (d; C-3), 43.63 (d; C-2), 36.38 (t; C-5), 32.22 (t; $\underline{C}H_2CH_2OH$).

| | | | |
|---|---|---|---|
| $C_{22}H_{28}O_4$ (356.5) | Ber. | C 74.13 | H 7.92 |
| | Gef. | C 74.40 | H 7.79 |

<u>(1R,2R,3R,4R)-1,4-Dibenzyloxy-3-hydroxymethyl-2-(2-p-phenylbenzoyloxyethyl)-cyclopentan</u> (**14**):

Zu 6.7 g (18.8 mmol) **13** in 8 ml Pyridin werden unter Rühren 5 g (23.1 mmol) p-Phenylbenzoylchlorid innerhalb von 1 h zugegeben. Die Reaktionsmischung wird 2 1/2 h bei Raumtemp. gerührt und mit 100 ml Ethylacetat versetzt. Die Lösung wird dreimal mit 50 ml Wasser ausgeschüttelt. Die organische Phase wird mit wasserfreiem Natriumsulfat getrocknet und i.Vak. bis zur Trockne eingedampft. Die Reaktionsprodukte werden säulenchromatographisch getrennt. Mit Toluol/Aceton (98:2) werden die Fraktionen 1-3 und mit Toluol/Aceton (7:3) wird die Fraktion 4 erhalten. Die Fraktionen 1 und 3 enthalten die möglichen anderen Ester, die mit Kaliumcarbonat in Methanol wieder zu **13** verseift werden. Die Fraktion 4 enthält noch etwas Ausgangsmaterial **13**.

<u>Fraktion 2:</u> **14** als farbloses Oel. Ausb. 3.4 g (34 %), $\underline{R_F}$ = 0.48 (Toluol/Aceton 8:2). - $^1$H-NMR (300 MHz, $CDCl_3$): $\delta$ = 8.10 - 7.24 (19H, Aromaten-H), 4.60 - 4.44 (4H, $OC\underline{H}_2C_6H_5$, 4,49 - 4.29 (m; 3H, 4-H, $CH_2C\underline{H}_2$OpPB), 3.93

($m_c$; 1H, 1-H), 3.83 (d; *J* = 6.19 Hz; 2H, $C\underline{H}_2OH$), 2.55 ($m_c$; 1H, 3-H), 2.24 - 2.04 (m; 3H, 5-H, 2-H), 2.01 - 1.91 (m; 2H, $C\underline{H}_2CH_2OpPB$). - $^{13}$C-NMR (20 MHz, $CDCl_3$): $\delta$ = 166.5 (s; C=O), 145.62 - 127.05 (C-Aromaten), 82.88 (d; C-4), 80.74 (d; C-1), 71.85 (t; $O\underline{C}H_2C_6H_5$), 71.72 (t; $O\underline{C}H_2C_6H_5$), 64.15 (t; $CH_2\underline{C}H_2OpPB$), 60.05 (t; $\underline{C}H_2OH$), 45.58 (d; C-3), 43.21 (d; C-2), 36.75 (t; C-5), 28.37 (t; $\underline{C}H_2CH_2OpPB$).

| | | | |
|---|---|---|---|
| $C_{35}H_{36}O_5$ (535.7) | Ber. | C 78.33 | H 6.76 |
| | Gef. | C 78.14 | H 6.73 |

<u>2-Decarboxy-15-dehydro-9,11-di-O-benzyl-6-(p-phenylbenzolyloxy)-2,3,4,5-tetranor-PGF$_{1\beta}$</u> (4a) <u>und sein C-12-Epimeres 4:</u>

2.9 g (13.5 mmol) Pyridiniumchlorochromat werden mit 13.5 g Aluminiumoxid (neutral) in einer Reibschale gut vermischt und in 28 ml wasserfreiem Dichlormethan suspendiert. Man rührt 2 h und gibt 3.4 g (6.35 mmol) 14 in 30 ml wasserfreiem Dichlormethan zu. Nach 3 h Rühren ist die Reaktion beendet. Man verdünnt mit 60 ml wasserfreiem Diethylether und dekantiert die überstehende Lösung ab. Der Rückstand wird noch achtmal mit je 20 ml Diethylether versetzt, aufgerührt und abdekantiert. Die vereinigten Etherlösungen werden über $Mg_2SiO_4$ abgesaugt. Man spült 300 ml Ether nach und dampft die Etherlösung i.Vak. zur Trockne ein. Ausb. an 15/15a: 3.1 g.
Zu einer Suspension von 225 mg (7.5 mmol) Natriumhydrid (80 % in Weißöl) in 60 ml wasserfreiem Dimethoxyethan werden bei Raumtemp. in einer mehrmals ausgeflämmten Apparatur unter Stickstoffatmosphäre 1.625 ml (7.8 mmol) 2-Oxoheptylphosphonsäuredimethylester gegeben. Man läßt 1 h rühren und tropft innerhalb von wenigen min 2.65 g (4.96 mmol) 15/15a in 30 ml Dimethoxyethan zu. Nach 4 h Rühren bei Raumtemp. wird der Ueberschuß des Phosphoniumsalzes mit 1.2 ml Eisessig zerstört, die Reaktionsmischung mit 100 ml Wasser verdünnt und dreimal mit je 100 ml Ethylacetat ausgeschüttelt. Nach Trocknung des Ethylacetats mit wasserfreiem Natriumsulfat wird das Lösungsmittel i.Vak. abgedampft und der Rückstand säulenchromatographisch mit Cyclohexan/Ethylacetat (95:5) als Elutionsmittel getrennt.

Fraktion 1: 4 als farbloses Oel Ausb. 1.6 g (51 %). Fraktion 2: 4a kristallisiert aus Cyclohexan, Ausb. 0.9 g (29 %), Schmp. 89 - 90°C (Cyclohexan), $R_F$ = 0.33 (Toluol/Aceton 8:2), $[\alpha]_{589}^{20}$ = +51° (c = 1 g in 100 ml Chloroform). - IR (KBr): 1720 (C=O), 1665 (C=O), 1610 (C=C), 980 $cm^{-1}$ (C=C, E-ständig). - $^{1}$H-NMR (300 MHz, $CDCl_3$): $\delta$ = 8.08 - 7.24 (19H, Aromaten-H), 6.77 (dd; $J_{13/14}$ = 15.9 Hz, $J_{12/13}$ = 8.8 Hz; 1H, 13-H), 6.19 (d; $J_{13/14}$ = 15.9 Hz; 1H, 14-H), 4.49 (4H, $OCH_2C_6H_5$), 4.39 ($m_c$; 2H, 6-H), 3.97 (q; 1H, 11-H), 3.90 (q; 1H, 9-H), 2.46 (t; 2H, 16-H), 2.44 ($m_c$; 1H, 12-H), 2.20 - 1.87 (m; 5H, 8-H, 7-H, 10-H), 1.58 ($m_c$; 2H, 17-H), 1.27 ($m_c$; 4H, 18-H, 19-H), 0.86 (t; 3H, 20-H), - $^{13}$CNMR (75.46 MHz, $CDCl_3$, Tab.4.).

| | | | |
|---|---|---|---|
| $C_{42}H_{46}O_5$ (630.8) | Ber. | C 79.9 | H 7.4 |
| | Gef. | C 80.14 | H 7.62 |

Die weiteren Reaktionen zu 11a und 4a werden analog - wie bei der Synthese des 12-epi-$PGF_{2\beta}$ (11) beschrieben - ausgeführt. Eine quantitative Trennung der C-15-Epimeren 5a und (15R)-5a konnte säulenchromatographisch nicht erreicht werden, so daß die Trennung besser auf der Stufe der Verbindungen 10a / (15R)-10a erfolgt.

2-Decarboxy-9,11-di-O-benzyl-6-(p-phenylbenzoyloxy)-2,3,4,5-tetranor $PGF_{1\beta}$ (5a):

Farbloses Oel. $R_F$ = 0.58 (Toluol/Aceton 8:2). - IR (Film): 3430 (OH), 1720 (C=O), 970 (C=C) E-ständig). - $^{1}$H-NMR (300 MHz, $CDCl_3$): $\delta$ = 8.10 - 7.24 (19H, Aromaten-H), 5.63 ($m_c$; 2H, 13-H, 14-H), 4.56 - 4.43 (4H, $OCH_2C_6H_5$), 4.49 ($m_c$; 1H, 6-$H_A$), 4.34 ($m_c$; 1H, 6-$H_B$), 4.05 ($m_c$; 1H, 15-H), 3.86 ($m_c$; 2H, 9-H, 11-H), 2.28 ($m_c$; 1H, 12-H), 2.10 - 1.85 (m; 5H, 8-H, 7-H, 10-H), 1.65 (verbreitertes s; 1H, OH), 1.48 ($m_c$; 2H, 16-H), 1.35 - 1.15 (m; 6H, 17-H, 18-H, 19-H), 0.82 ($m_c$; 3H, 20-H). - $^{13}$C-NMR (75.46 MHz,

$CDCl_3$, Tab.4).

$C_{42}H_{48}O_5$ (632.8) Ber. C 79.71 H 7.65
Gef. C 79.76 H 7.72

(15R)-5a: Farbloses Oel. $R_F$ = 0.59 (Toluol/Aceton 8:2). - IR (Film): 3440 (OH), 1720 (C=O), 970 $cm^{-1}$ (C=C, E-ständig). - $^1H$-NMR (300 MHz, $CDCl_3$): $\delta$ = 8.10 - 7.24 (19H, Aromaten-H), 5.63 ($m_c$; $J_{13/14}$ = 15.3 Hz; 1H, 14-H), 5.33 ($m_c$; $J_{13/14}$ = 15.3 Hz, 1H, 13-H), 4.56 - 4.43 (m; 5H, 6-$H_A$, $OCH_2C_6H_5$), 4.35 - 4.27 (m; 1H, 6-$H_B$), 3.99 ($m_c$; 1H, 15-H), 3.82 ($m_c$; 2H, 9-H, 11-H), 2.28 ($m_c$; 1H, 12-H), 2.1 - 1.87 (m; 5H, 7-H, 8-H, 10-H), 1.7 (verbreitertes s; 1H, OH), 1.60 - 1.40 (m; 2H, 16-H), 1.40 - 1.20 (m; 6H, 17-H, 18-H, 19-H), 0.86 ($m_c$; 3H, 20-H). - $^{13}C$-NMR (75.46 MHz, $CDCl_3$, Tab.4).

$C_{42}H_{48}O_5$ (632.8) Ber. C 79.71 H 7.65
Gef. C 79.53 H 7.85

9.11-Di-O-benzyl-$PGF_{2\beta}$ (10a): Farbloses Oel. Ausb. 230 mg (30 %). $R_F$ = 0.35 (Toluol/Ethylacetat/Methanol/Eisessig 80:20:5:1). - IR (Film): 3400 (OH), 1710 (C=O), 970 $cm^{-1}$ (C=C, E-ständig). - $^1H$-NMR (300 MHz, $CDCl_3$, H/D-Austausch): $\delta$ = 7.3 ($m_c$; 10H, Aromaten-H), 5.59 ($m_c$; $J_{13/14}$ = 15.5 Hz; 2H, 13-H, 14-H), 5.50 - 5.30 (m; 2H, 5-H,6-H), 4.50 - 4.39 (m; 4H, $OCH_2C_6H_5$), 4.09 (q; 1H, 15-H), 3.86 (q; 1H, 11-H), 3.76 ($m_c$; 1H, 9-H), 2.30 (t; 2H, 2-H), 2.34 - 2.25 (m; 1H, 12-H), 2.23 - 1.77 (m; 7H, 4-H, 7-H, 8-H, 10-H), 1.65 ($m_c$; 2H, 3-H), 1.51 ($m_c$; 2H, 16-H), 1.28 ($m_c$; 6H, 17-H, 18-H, 19-H), 0.86 ($m_c$; 3H, 20-H). - $^{13}C$-NMR (75.46 MHz, $CDCl_3$, Tab. 4).

$C_{34}H_{46}O_5$ (534.7) Ber. C 76.37 H 8.67
Gef. C 76.10 H 8.67

(15R)-9-11-Di-O-benzyl-$PGF_{2\beta}$ ((15R)-10a):
Farbloses Oel. Ausb. 230 mg (30 %), $R_F$ = 0.38 (Toluol/Ethylacetat/Methanol/Eisessig 80:20:5:1). IR (Film): 3400 (OH), 1710 (C=O), 970 $cm^{-1}$ (C=C, E-ständig). -

$^{1}$H-NMR (300 MHz, $CDCl_3$, H/D-Austausch): $\delta$ = 7.3 ($m_c$; 10H, Aromaten-H) 5.60 ($m_c$; 2H, 13-H, 14-H), 5.60 - 5.35 (m; 2H, 5-H, 6-H), 4.55 - 4.38 (4H, $OCH_2C_6H_5$), 4.10 ($m_c$; 1H, 15-H), 3.85 ($m_c$; 1H, 11-H), 3.77 ($m_c$; 1H, 9-H), 2.42 - 2.28 (m; 3H, 2-H, 12-H), 2.19 - 1.43 (m; 11H, 3-H, 4-H, 7-H, 8-H, 10-H, 16-H), 1.25 (m; 6H, 17-H, 18-H, 19-H), 0.86 ($m_c$; 3H, 20-H). - $^{13}$C-NMR (75.46 MHz, $CDCl_3$, Tab. 4 ).

| | | | |
|---|---|---|---|
| $C_{34}H_{46}O_5$ (534.7) | Ber. | C 76.37 | H 8.67 |
| | Gef. | C 76.35 | H 8.72 |

PGF$_{2\beta}$ (11a): Farblose Kristalle aus Ethylacetat. Schmp. 95°C, Ausb. 120 mg (79 %), $R_F$ = 0.18 (Ethylacetat/Isopropanol/Eisessig 80:10:2), $[\alpha]^{20}_{589}$ = -5° (c = 1 g in 100 ml Methanol). - IR (KBr): 3460 (OH), 3270 (OH), 1710 (C=O), 970 $cm^{-1}$ (C=C, E-ständig). - $^{1}$H-NMR (300 MHz, $CDCl_3/CD_3OD$ 9:1): $\delta$ = 5.47 ($m_c$; 4H, 5-H, 6-H, 13-H, 14-H), 4.02 - 3.85 (m; 3H, 9-H, 11-H, 15-H), 2.31 (t; J = 7.3 Hz; 2H, 2-H), 2.26 - 1.44 (m; 10H, 4-H, 7-H, 8-H, 10-H, 12-H, 16-H), 1.68 (quint; 2H, 3-H), 1.28 ($m_c$; 6H, 17-H, 18-H, 19-H), 0.86 ($m_c$; 3H, 20-H). - $^{13}$C-NMR (75.46 MHz, $CDCl_3/CD_3OD$ 9 : 1, Tab. 1).

| | | | |
|---|---|---|---|
| $C_{20}H_{34}O_5$ (354.5) | Ber. | C 67.77 | H 9.67 |
| | Gef. | C 67.77 | H 9.71 |

(15R)-PGF$_{2\beta}$ ((15R)-11a): Farblose Kristalle aus Ethylacetat. Ausb. 72 %, Schmp. 75 - 76°C, $R_F$ = 0.29 (Ethylacetat/Isopropanol/Eisessig 80:10:2), $[\alpha]^{20}_{589}$ = -11.6 (c = 1 g in 100 ml Methanol/. - IR (KBr): 3380 (OH), 1710 (C=O), 970 $cm^{-1}$ (C=C, E-ständig). - $^{1}$H-NMR (300 MHz, $CDCl_3/CD_3OD$ 9:1): $\delta$ = 5.58 ($m_c$; 2H, 13-H, 14-H), 5.46 ($m_c$; 2H, 5-H-6-H), 4.0 ($m_c$; 3H, 9-H, 11-H, 15-H), 2.32 (t; 2H, 2-H), 2.32 - 2.23 (m; 1H, 12-H), 2.15 - 1.38 (m; 9H, 4-H, 7-H, 8-H, 10-H, 16-H), 1.68 (quint; 2H, 3-H), 1.30 ($m_c$; 6H, 17-H, 18-H, 19-H), 0.89 ($m_c$; 3H, 20-H). - $^{13}$C-NMR (75.46 MHz, $CDCl_3/CD_3OD$ 9:1), Tab. 2).

$C_{20}H_{34}O_5$ (354.5) Ber. C 67.77 H 9.67
Gef. C 67.22 H 9.41

Beispiel 4:

Synthese der 15-Methyl-12-epi-$PGF_{2\beta}$-Diastereomeren aus **2** ($R^1$=$R^2$=p-Phenylbenzoyl)

9,11-Bis(-O-p-phenylbenzoyl)-2-decarboxy-15-dehydro-6-hydroxy-2,3,4,5-tetranor-12-epi-$PGF_{1\beta}$ (**16**):

Unter Argon werden 0.34 g (11.2 mmol) Natriumhydrid (80 % in Weißöl) in 400 ml wasserfreiem THF suspendiert und unter Rühren 3.2 g (14.6 mmol) (2-Oxoheptyl)-phosphonsäuredimethylester zugegeben. Man läßt 1 h bei Raumtemp. und 1 h bei 30°C rühren. Unter Einleiten eines schwachen Argonstroms gibt man 6.0 g (11.2 mmol) feingepulvertes **2** ($R^1$=$R^2$=p-Phenylbenzoyl) hinzu und läßt 15 h bei Raumtemp. rühren. Unter Eiskühlung fügt man 20 ml 0.1 M Essigsäure und 150 ml Wasser zu, extrahiert 3x mit je 100 ml Ethylacetat, trocknet die vereinigten Extrakte über wasserfreiem $Na_2SO_4$ und dampft mit wenig Celite i.Vak. zur Trockne ein. Der Rückstand wird auf eine Kieselsäure-Drucksäule (80 cm x 2 cm) gegeben und nacheinander mit 1 l Dichlormethan, 1 l Dichlormethan/Aceton (98:2) und 1 l Dichlormethan/Aceton (95:5) eluiert. **16** wird aus Toluol/n-Pentan umkristallisiert. Ausb. 2.1 g (30 %), Schmp. 138 - 139°C, $R_F$ = 0.60 (Dichlormethan/Aceton 9:1), $[\alpha]^{20}_{589}$ = -67.8 (c = 1.0 g in 100 ml $CHCl_3$. - $^1H$-NMR (300 MHz, $CDCl_3$): $\delta$ = 8.15 - 8.03, 7.71 - 7.60 und 7.51 - 7.36 (m; 18H, Aromaten-H), 6.87 (dd; $J_{13/14}$ = 15.5 Hz, $J_{12/13}$ = 10.0 Hz; 1H, 13-H), 6.23 (d; $J_{13/14}$ = 15.5 Hz; 1H, 14-H), 5.69 ($m_c$; 1H, 11-H), 5.49 ($m_c$; 1H, 9-H), 3.90 - 3.73 (m; 2H, 6-H), 3.45 ($m_c$; $J_{8/12}$ = 7.0 Hz; 1H, 12-H), 2.73 - 2.62 (m; 2H, 8-H und 10-H), 2.56 ($m_c$; $^2J$ = 15.3 Hz; 1H, 10-H), 2.44 ($m_c$; $^2J$ = 15.8 Hz; 2H, 16-H), 2.18 (s; 1H, OH), 1.85 ($m_c$; 2H, 7-H), 1.52 ($m_c$; 2H, 17-H), 1.29 - 1.17 (m; 4H, 18-H und 19-H), 0.82 (t; 3H, 20-H). - $^{13}C$-NMR (75.46 MHz, $C_6D_6$, Tab. 3).

1$C_{41}H_{42}O_6$ (630.8) Ber. C 78.07 H 6.71
Gef. C 78.15 H 6.77

Als Nebenprodukt entsteht mit 18 % Ausbeute (1R,6R,7R,9R)-(+)-7,9-Bis(p-phenylbenzoyloxy)-2-(2'-oxoheptyl)-3-oxa-bicyclo[4.3.0]nonan ($R_F$ = 0.79).

(15R)-und (15S)-9,11-Bis(-O-p-phenylbenzoyl)-2-decarboxy-6-hyddroxy-15-methyl-2,3,4,5-tetranor-12-epi-$PGF_{2\beta}$ [(15R)-17 und 17]:

Die Ausbeute an C-15-Epimeren (15R)-3 und 3 wird erhöht, wenn man 16 nicht isoliert, sondern zu der Reaktionslösung nachdem sie 15 h gerührt wurde (s.o.), zwischen -75° und -72°C (Methanol/Trockeneis) 120 ml einer 2 M $CH_3MgBr$-Lösung in Ether tropft. Man läßt 2 h bei -75°C unter Argon rühren und tropft bei dieser Temp. langsam 180 ml einer gesättigten $NH_4Cl$-Lösung zu. Die entstandene Suspension läßt man auf Raumtemp. erwärmen und fügt so viel Wasser zu, bis sich der Niederschlag gerade löst. Es wird dreimal mit je 200 ml Ethylacetat extrahiert. Die vereinigten Ethylacetatphasen werden über wasserfreiem $Na_2SO_4$ getrocknet und i.Vak. eingedampft. Der Rückstand wird aus Ethanol umkristallisiert. Die Kristalle werden in Dichlormethan gelöst, auf Celite aufgezogen und auf eine Kieselsäure-Drucksäule (80 cm x 2 cm) gegeben, die nacheinander mit 1 l Dichlormethan, 1 l Dichlormethan/Aceton (98:2) und 3 l Dichlormethan/Aceton (95:5) eluiert wird. (15R)-17 und 17 sind nach fünf Säulendurchgängen quantitativ getrennt.

(15R)-17: Farblose Kristalle aus Ethanol. Ausb. 1.0 g (14 %, bez. auf 2), Schmp. 177 - 178°C, $R_F$ = 0.29 (Dichlormethan/Aceton 9:1), $[\alpha]_{589}^{20}$ = -34.2 (c = 1.0 g in 100 ml $CHCl_3$). - $^1$H-NMR (300 MHz, $CDCl_3$): $\delta$ = 8.13 - 8.05, 7.70 - 7.60 und 7.51 - 7.35 (m; 18 H, Aromaten-H), 5.72 - 5.56 (m; 3H, 11-H, 13-H und 14-H), 5.42 ($m_c$; 1H, 9-H), 3.88 - 3.72 (m; 2H, 6-H), 3.34 ($m_c$; $J_{8/12}$ = 6.6 Hz; 1H, 12-H), 2.70 - 2.41 (m; 3H, 8-H und 10-H), 2.17 (s; 1H, OH), 1.95 - 1.74 (m; 2H, 7-H), 1.68 (s; 1H, OH),

1.42 - 1.33 (m; 2H, 16-H), 1.27 - 0.98 (m; 6H, 17-H, 18-H, 19-H), 1.20 (s; 3H, $-CH_3$ an C-15), 0.75 (t; 3H, 20-H). - $^{13}$C-NMR (75.46 MHz, $CDCl_3$, Tab. 3).

$C_{42}H_{46}O_6$ (646.8) Ber. C 77.99 H 7.17
Gef. C 78.22 H 7.20

17: Farblose Kristalle aus Ethanol. Ausb. 1.6 g (22 % bez. auf 2), Schmp. 159 - 160°C, $R_F$ = 0.22 (Dichlormethan/Aceton 9:1). $[\alpha]_{589}^{20}$ = -30.1 (c = 1.1 g in 100 ml $CHCl_3$).- $^1$H-NMR (300 MHz, $CDCl_3$): $\delta$ = 8.13 - 8.05, 7.70 - 7.60 und 7.51 - 7.35 (m; 18H, Aromaten-H), 5.71 - 5.55 (m; 3H, 11-H, 13-H und 14-H), 5.41 ($m_c$; 1H, 9-H), 3.85 - 3.69 (m; 2H, 6-H), 3.33 ($m_c$; $J_{8/12}$ = 7.1 Hz; 1H, 12-H), 2.69 - 2.41 (m; 3H, 8-H und 10-H), 2.25 (s; 1H, OH), 1.91 - 1.77 (m; 2H, 7-H), 1.52 (s; 1H, OH), 1.42 - 1.33 (m; 2H, 16-H), 1.28 - 1.02 (m; 6H, 17-H, 18-H und 19-H), 1.12 (s; 3H, $-CH_3$ an C-15), 0.80 (t; 3H, 20-H). - $^{13}$C-NMR (75.46 MHz, $CDCl_3$, Tab. 3).

$C_{42}H_{46}O_6$ (646.8) Ber. C 77.99 H 7.17
Gef. C 77.93 H 7.25

(15R)- und (15S)-9,11-Bis(-O-p-phenylbenzoyl)-2-decarboxy-15-methyl-6-oxo-2,3,4,5-tetranor-12-epi-$PGF_{1\beta}$ [(15R)-18 und 18]:

6.0 g $Al_2O_3$ und 1.5 g (7.0 mmol) Pyridinium-chlorochromat werden in 70 ml wasserfreiem Dichlormethan suspendiert und 30 min bei Raumtemp. gerührt. Nach Zugabe von 0.5 g (0.77 mmol) 17 bzw. (15R)-17 läßt man 2 h bei 15-20°C rühren. Die dunkelbraune Suspension wird über $Mg_2O_8Si_3 \cdot 2H_2O$ filtriert, mit Dichlormethan gewaschen und i. Vak. zur Trockne eingedampft. Der Rückstand von 18 bzw. (15R)-18 wird aus Ethanol umkristallisiert.

(15R)-18 : Ausb. 0.34 g (68 %), Schmp. 185 - 186°C, $R_F$ = 0.65 (Dichlormethan/Aceton 9:1), $[\alpha]_{589}^{20}$ = -28.1 (c = 0.9 g in 100 ml $CHCl_3$). - $^1$H-NMR (300 MHz, $CDCl_3$): $\delta$ = 9.79 (s; 1H, 6-H), 8.14 - 8.05, 7.70 - 7.59, 7.50 - 7.37 (m; 18H, Aromaten-H), 5.67 - 5.54 (m; $J_{13/14}$=15.0 Hz;

3H, 11-H, 13-H u. 14-H), 5.35 ($m_c$; 1H, 9-H), 3.48 ($m_c$; $J_{8/12}$ = 6.2 Hz; 1H, 12-H), 2.95 ($m_c$; 1H, 7-H), 2.77 ($m_c$; $^2J$ = 7.5 Hz; 2H, 7-H und 8-H), 2.66 ($m_c$; $^2J$ = 15.0 Hz, $^3J$ = 6.6 Hz; 1H, 10-H), 2.42 ($m_c$; $^2J$ = 15.0 Hz, $^3J$ = 4.9 Hz; 1H, 10-H), 1.66 (s; 1H, OH), 1.40 - 1.32 (m; 2H, 16-H), 1.21 (s; 3H, $-CH_3$ an C-15), 1.17 - 1.00 (m; 6H, 17-H, 18-H und 19-H), 0.75 (t; 3H, 20-H). - $^{13}$C-NMR (75.46 MHz, Tab. 3).

$C_{42}H_{44}O_6$ (644.8) Ber. C 78.23 H 6.88
Gef. C 78.31 H 6.87

18: Ausb. 0.33 g (66 %), Schmp. 164 - 165°C, $R_F$ = 0.70 (Dichlormethan/Aceton 9:1), $[\alpha]_{589}^{20}$ = -25.3 (c = 0.9 g in 100 ml $CHCl_3$). - $^1$H-NMR (300 MHz, $CDCl_3$): $\delta$ = 9.79 (s; 1H, 6-H), 8.14 - 8.05, 7.70 - 7.59 und 7.50 - 7.37 (m; 18H, Aromaten-H), 5.67 - 5.52 (m; $J_{13/14}$ = 15.5 Hz; 3H, 11-H, 13-H und 14-H), 5.36 ($m_c$; 1H, 9-H), 3.48 ($m_c$; $J_{8/12}$ = 6.6 Hz; 1H, 12-H), 2.94 ($m_c$; $^2J$ = 7.1 Hz; 1H, 7-H), 2.76 ($m_c$; 2H, 7-H und 8-H), 2.64 ($m_c$; $^2J$ = 15.0 Hz, $^3J$ = 8.8 Hz; 1H, 10-H), 2.42 ($m_c$; $^2J$ = 15.0 Hz, $^3J$ = 8.4 Hz; 1H, 10-H), 1.63 (s; 1H, OH), 1.45 - 1.32 (m; 2H, 16-H), 1.30 - 1.12 (m; 6H, 17-H, 18-H und 19-H), 1.10 (s; 3H, $-CH_3$ an C-15), 0.82 (t; 3H, 20-H). $^{13}$C-NMR (75.46 MHz, $CDCl_3$, Tab. 3).

$C_{42}H_{44}O_6$ (644.8) Ber. C 78.23 H 6.88
Gef. C 78.37 H 7.05

(15R)- und (15S)-9,11-Bis(-O-p-phenylbenzoyl)-15-methyl-12-epi-PGF$_{2\beta}$ [(15R)-19 und 19]:

Unter Argon suspendiert man 1.33 g (3.0 mmol) 4-Carboxybutyl-triphenylphosphoniumbromid in 150 ml wasserfreiem THF, gibt 0.67 g (6.0 mmol) Kalium-tert.-butylat zu und rührt 30 min bei Raumtemp.. Nach Abkühlen auf -78°C wird 1.0 g (1.55 mmol) 18 bzw. (15R)-18 zugegeben. Die gelborange gefärbte Suspension wird 2 h bei -78°C gerührt und innerhalb von 1 h auf Raumtemp. erwärmt. Die Reaktionslösung wird nacheinander mit 30 ml 1 M Phosphatpuffer (pH = 4.5) und 30 ml Wasser versetzt, dreimal mit

je 50 ml Ethylacetat ausgeschüttelt, und die organischen Phasen werden über wasserfreiem $Na_2SO_4$ getrocknet. Nach dem Abdampfen des Lösungsmittels i. Vak. wird der Rückstand von **19** bzw. (15R)-**19** aus Methanol umkristallisiert.

(15R)-**19** : Ausb. 0.8 g (71 %), Schmp. 121-123°C, $R_F$ = 0.39 (Dichlormethan/Aceton 9:1), $[\alpha]_{589}^{20}$ = -27.9 ($c$ = 1,0 g in 100 ml $CHCl_3$). - $^1$H-NMR (300 MHz, $CDCl_3$): $\delta$= 8.12 - 8.04, 7.68 - 7.57 und 7.50 - 7.34 (m; 18H, Aromaten-H), 5.75 - 5.35 (m; $J_{5/6}$ = 11.1 Hz, $J_{12/13}$ = 8.8 Hz, $J_{13/14}$ = 15.5 Hz; 6H, 5-H, 6-H, 9-H, 11-H, 13-H und 14-H), 3.34 ($m_c$; $J_{8/12}$ = 6.6 Hz; 1H, 12-H), 2.67 ($m_c$; $^2J$ = 15.0 Hz; 1H, 10-H), 2.50 - 2.20 (m; 6H, 2-H, 7-H, 8-H und 10-H), 2.10 ($m_c$; 2H, 4-H), 1.62 ($m_c$; 2H, 3-H), 1.42 - 1.29 (m; 2H, 16-H), 1.21 (s; 3H, $-CH_3$ an C-15), 1.18 - 0.92 (m; 6H, 17-H, 18-H und 19-H), 0.70 (t; 3H, 20-H).- $^{13}$C-NMR $CDCl_3$, Tab. 3).

| | | | |
|---|---|---|---|
| $C_{47}H_{52}O_7$ (728.9) | Ber. | C 77.45 | H 7.19 |
| | Gef. | C 77.59 | H 7.28 |

**19**: Ausb. 0.8 g (71 %), Schmp. 162 - 164°C, $R_F$ = 0.41 (Dichlormethan/Aceton 9:1), $[\alpha]_{589}^{20}$ = -25.8 ($c$ = 1.0 g in 100 ml $CHCl_3$). - $^1$H-NMR (300 MHz, $CDCl_3$, H/D-Austausch): $\delta$ = 8.11 - 8.04, 7.70 - 7.61 und 7.50 - 7.35 (m; 18H, Aromaten-H), 5.73 - 5.30 (m; $J_{5/6}$ = 11.1 Hz, $J_{12/13}$ = 9.3 Hz, $J_{13/14}$ = 15.5 Hz; 6H, 5-H, 6-H, 9-H, 11-H, 13-H und 14-H), 3.33 ($m_c$; $J_{8/12}$ = 6.2 Hz; 1H, 12-H), 2.66 ($m_c$; $^2J$ = 15.2 Hz; 1H, 10-H), 2.49 - 2.23 (m; 6H, 2-H, 7-H, 8-H und 10-H), 2.11 ($m_c$; 2H, 4-H), 1.64 ($m_c$; 2H, 3-H), 1.48 - 1.35 (m; 2H, 16-H), 1.26 - 1.05 (m; 9H, $-CH_3$ an C-15, 17-H, 18-H und 19-H), 0.79 (t; 3H. 20-H). - $^{13}$C-NMR (75.46 MHz, $CDCl_3$, Tab. 3).

| | | | |
|---|---|---|---|
| $C_{47}H_{52}O_7$ (728.9) | Ber. | C 77.45 | H 7.19 |
| | Gef. | C 77.50 | H 7.27 |

(15R)- und(15S)-15-Methyl-epi-PGF$_{2\beta}$ [(15R)-20 und 20]: 0.5 g (0.67 mmol) 19 bzw. (15R)-19 werden in 25 ml wasserfreiem Methanol suspensiert und mit 0.56 g (4.02 mmol) $K_2CO_3$ (p.a.) versetzt. Nach 18 h Rühren bei 35°C wird die Reaktionslösung mit 0.1 M Essigsäure neutralisiert, mit 10 ml 10proz. NaCl-Lösung verdünnt und fünfmal mit je 15 ml Chloroform extrahiert. Die Extrakte werden über wasserfreiem $Na_2SO_4$ getrocknet und mit wenig Celite i.Vak. zur Trockne eingedampft. Der Rückstand wird über eine Kieselsäure-Drucksäule (60 cm x 1.5 cm) mit 1 l Chloroform/Methanol (95:5) gereinigt. 20 und (15R)-20 kristallisieren aus Ethylacetat/n-Pentan (1:1).

(15R)-20: Ausb. 0.19 g (77 %), Schmp. 111 - 112°C, $R_F$ = 0.15 ($CHCl_3$/Methanol 9:1). $[\alpha]^{20}_{589}$ = -19.6 (c = 1.0 g in 100 ml $CHCl_3$). - $^1H$-NMR (300 MHz, $CDCl_3$, H/D-Austausch): $\delta$ = 5.67 - 5.38 (m; $J_{5/6}$ = 10.6 Hz, $J_{12/13}$ = 9.7 Hz, $J_{13/14}$ = 15.5 Hz; 4H, 5-H, 6-H, 13-H und 14-H), 4.34 ($m_c$; 1H, 11-H), 4.13 ($m_c$; 1H, 9-H), 2.85 ($m_c$; $J_{8/12}$ = 6.6 Hz; 1H, 12-H), 2.35 - 2.25 (m; 3H, 2-H und 7-H), 2.21 - 1.83 (m; 6H, 4-H, 7-H, 8-H und 10-H), 1.75 - 1.63 (m; 2H, 3-H), 1.61 - 1.50 (m; 2H, 16-H), 1.38 - 1.20 (m; 6H, 17-H, 18-H und 19-H), 1.28 (s; 3H, -C$H_3$ an C-15), 0.88 (t; 3H, 20-H). - $^{13}C$-NMR (75.46 MHz, $CDCl_3$, Tab. 3).

| | | | |
|---|---|---|---|
| $C_{21}H_{36}O_5$ (368.5) | Ber. | C 68.45 | H 9.85 |
| | Gef. | C 68.61 | H 9.66 |

20: Ausb. 0.20 g (81 %), Schmp. 84°C, $R_F$ = 0.15 (Chloroform/Methanol 9:1), $[\alpha]^{20}_{589}$ = +4.9 (c = 1.0 g in 100 ml $CHCl_3$). - $^1H$-NMR (300 MHz, $CDCl_3$, H/D-Austausch): $\delta$ = 5.65 - 5.33 (m; $J_{5/6}$ = 11.1 Hz, $J_{12/13}$ = 10.2 Hz, $J_{13/14}$ = 15.5 Hz; 4H, 5-H, 6-H, 13-H und 14-H), 4.31 ($m_c$; 1H, 11-H), 4.09 - 4.03 (m; 1H, 9-H), 2.84 ($m_c$; $J_{8/12}$ = 6.2 Hz; 1H, 12-H), 2.35 - 2.25 (m; 3H, 2-H und 7-H), 2.17 - 1.91 (m; 5H, 4-H, 7-H und 10-H), 1.83 (m; 1H, 8-H), 1.72 - 1.62 (m; 2H, 3-H), 1.56 - 1.44 (m; 2H, 16-H), 1.44 - 1.22 (m; 9H, -C$H_3$ an C-15, 17-H, 18-H und 19-H) 0.88

(t; 3H, 20-H). - $^{13}$C-NMR (75.46 MHz, $CDCl_3$, Tab. 3).

$C_{21}H_{36}O_5$ (368.5) Ber. C 68.45 H 9.85
Gef. C 68.35 H 9.71

(15R)- und (15S)-15-Methyl-12-epi-$PGF_{2\beta}$-methylester [(15R)-21 und 21]:

20 bzw. (15R)-20 werden mit einer etherischen Diazomethanlösung verestert.

(15R)-21: Farbloses Oel, Ausb. 91 %, $R_F$ = 0.25 (Chloroform/Methanol 9:1). $[\alpha]^{20}_{589}$ = -30.2 ($c$ = 1.0 g in 100 ml $CHCl_3$). - $^1$H-NMR (300 MHz, $CDCl_3$, H/D-Austausch): $\delta$ = 5.68 - 5.34 (m; $J_{5/6}$ = 11.1 Hz, $J_{12/13}$ = 10.2 Hz, $J_{13/14}$ = 15.5 Hz; 4H, 5-H, 6-H, 13-H und 14-H), 4.35 ($m_c$; 1H, 11-H), 4.11 ($m_c$; 1H, 9-H), 3.67 (s; 3H, $-CO_2CH_3$), 2.82 ($m_c$; $J_{8/12}$ = 6.6 Hz; 1H, 12-H), 2.34 - 2.21 (m; 1H, 7-H), 2.32 (t; 2H, 2-H), 2.14 - 1.91 (m; 5H, 7-H und 10-H), 1.84 ($m_c$; 1H, 8-H), 1.67 ($m_c$; 2H, 3-H), 1.50 - 1.47 (m; 2H, 16-H), 1.38 - 1.20 (m; 9H, $-CH_3$ an C-15, 17-H, 18-H und 19-H), 0.88 (t; 3H, 20-H). - $^{13}$C-NMR (75.46 MHz, $CDCl_3$, Tab. 3).

$C_{22}H_{38}O_5$ (382.5) Ber. C 69.08 H 10.01
Gef. C 68.83 H 10.01

21: Farblose Kristalle aus Ethylacetat/n-Pentan. Aus. 88% Schmp. 56 - 57°C, $R_F$ = 0.25 (Chloroform/Methanol 9:1), $[\alpha]^{20}_{589}$ = -21.0 ($c$ = 1.0 g in 100 ml $CHCl_3$). - $^1$H-NMR (300 MHz, $CDCl_3$, H/D-Austausch): $\delta$= 5.67 - 5.35 (m; $J_{12/13}$ = 10.2 Hz, $J_{13/14}$ = 15.5 Hz; 4H, 5-H, 6-H, 13-H u. 14-H), 4.34 ($m_c$; 1H, 11-H), 4.12 ($m_c$; 1H, 9-H), 3.67 (s; 3H, $-CO_2CH_3$), 2.84 ($m_c$; $J_{8/12}$ = 6.6 Hz; 1H, 12-H), 2.34 - 2.23 (m; 1H, 7-H), 2.32 (t; 2H, 2-H), 2.15 - 1.93 (m; 5H, 4-H, 7-H und 10-H), 1.85 ($m_c$; 1H, 8-H), 1.68 ($m_c$; 2H, 3-H), 1.55 - 1.48 (m; 2H, 16-H), 1.38 - 1.22 (m; 9H, $-CH_3$ an C-15, 17-H, 18-H und 19-H), 0.87 (t; 3H, 20-H). - $^{13}$C-NMR (75.46 MHz, $CDCl_3$, Tab. 3).

$C_{22}H_{38}O_5$ (382.5) Ber. C 69.08 H 10.01
Gef. C 68.99 H 10.13

In den Tabellen 1 und 2 sind die $^{13}C$-chemischen Verschiebungen der erfindungsgemäß hergestellten Prostaglandin-Derivate 11, 11a und 12, jeweils mit 15S- und 15R-Konfiguration, angegeben. Der Vergleich der $^{13}C$-NMR-chemischen Verschiebungen von 11/11a zeigt, daß sich die σ-Werte der Kohlenstoffatome 6 bis 8 und 12 bis 14 am meisten unterscheiden. Vergleichbare Verschiebungsunterschiede sind auch von cis/trans-1,2-disubstituierten Cyclopentanen bekannt. In Tabelle 3 sind die $^{13}C$-NMR-chemischen Verschiebungen der Verbindungen 16 - 21 und ihrer (15R)-Epimeren aufgeführt. In Tabelle 4 sind die $^{13}C$-chemischen Verschiebungen für $C^1$ bis $C^{20}$ der bei der Synthese der erfindungsgemäßen Verbindungen isolierten Zwischenprodukte und der zum Konfigurationsbeweis hergestellten epimeren Zwischenprodukte angegeben.

Tab. 1 $^{13}C$-Chemische Verschiebungen der Prostaglandine mit 15S-Konfiguration

| C- | $PGF_{2\beta}$ (11a) | 12-epi-$PGF_{2\beta}$ (11) | 12-epi-$PGF_{2\beta}$-methylester (12) |
|---|---|---|---|
| 1 | 176.6 | 177.2 | 174.6 |
| 2 | 33.5 | 33.0 | 33.4 |
| 3 | 24.8 | 24.6 | 24.7 |
| 4 | 26.4 | 26.4 | 26.7 |
| 5 | 130.4 | 129.7 | 129.5 |
| 6 | 128.1 | 129.7 | 129.5 |
| 7 | 29.2 | 27.7 | 27.5 |
| 8 | 52.0 | 50.7 | 50.7 |
| 9 | 74.0 | 73.2 | 73.0 |
| 10 | 42.1 | 42.0 | 42.0 |
| 11 | 75.0 | 75.6 | 75.3 |
| 12 | 56.2 | 50.9 | 50.7 |
| 13 | 133.4 | 125.6 | 124.9 |
| 14 | 135.5 | 137.8 | 138.1 |
| 15 | 73.2 | 72.1 | 71.5 |
| 16 | 37.0 | 37.2 | 37.2 |
| 17 | 25.3 | 25.2 | 25.2 |
| 18 | 31.9 | 31.8 | 31.9 |
| 19 | 22.7 | 22.7 | 22.6 |
| 20 | 14.0 | 14.1 | 14.0 |

Tab. 2 $^{13}$C-Chemische Verschiebungen der Prostaglandine mit 15R-Konfiguration

| C- | 15R-PGF$_{2\beta}$ (15R)-11a | 15R-12-epi-PGF$_{2\beta}$ (15R)-11 | 15R-12-epi-PGF$_{2\beta}$ -methylester (15R)-12 |
|---|---|---|---|
| 1 | 176.6 | 176.9 | 174.4 |
| 2 | 33.4 | 33.4 | 33.5 |
| 3 | 24.8 | 24.8 | 24.9 |
| 4 | 26.5 | 26.6 | 26.8 |
| 5 | 130.4 | 129.5 | 129.6 |
| 6 | 128.2 | 129.2 | 129.3 |
| 7 | 29.2 | 27.5 | 27.7 |
| 8 | 52.3 | 50.7 | 50.8 |
| 9 | 74.2 | 73.2 | 73.1 |
| 10 | 42.4 | 41.6 | 42.1 |
| 11 | 75.1 | 75.2 | 75.5 |
| 12 | 55.7 | 50.7 | 50.9 |
| 13 | 131.7 | 128.0 | 127.8 |
| 14 | 135.0 | 137.9 | 138.3 |
| 15 | 72.3 | 72.8 | 72.9 |
| 16 | 37.1 | 36.7 | 37.0 |
| 17 | 25.3 | 25.3 | 25.3 |
| 18 | 31.9 | 31.8 | 31.8 |
| 19 | 22.7 | 22.7 | 22.7 |
| 20 | 14.1 | 14.1 | 14.1 |

Tab. 3 $^{13}$C-chemische Verschiebungen von 16 - 21 und ihrer (15R)-Epimeren in $CDCl_3$

| C- | 16 | (15R)-17 | 17 | (15R)-18 | 18 | (15R)-19 | 19 | (15R)-20 | 20 | (15R)-21 | 21 |
|---|---|---|---|---|---|---|---|---|---|---|---|
| 1 | - | - | - | - | - | 177.54 | 177.11 | 177.17 | 176.90 | 174.44 | 174.50 |
| 2 | - | - | - | - | - | 32.84 | 32.62 | 33.16 | 33.55 | 33.43 | 33.49 |
| 3 | - | - | - | - | - | 24.46 | 24.36 | 24.60 | 24.92 | 24.80 | 24.89 |
| 4 | - | - | - | - | - | 26.46 | 26.36 | 26.45 | 26.72 | 26.72 | 26.75 |
| 5 | - | - | - | - | - | 129.67 | 129.71 | 129.48 | 129.50 | 129.48 | 129.56 |
| 6 | 61.02 | 60.99 | 60.99 | 200.74 | 200.59 | 128.65 | 128.76 | 129.48 | 129.50 | 129.48 | 129.56 |
| 7 | 32.44 | 32.20 | 32.20 | 43.95 | 44.01 | 27.03 | 27.23 | 27.59 | 27.71 | 27.68 | 27.89 |
| 8 | 45.66 | 45.03 | 45.00 | 42.27 | 42.09 | 48.03 | 47.87 | 50.63 | 50.96 | 50.81 | 51.02 |
| 9 | 76.12 | 76.03 | 75.91 | 75.31 | 75.25 | 75,74 | 75,67 | 73.07 | 73.10 | 72.89 | 73.04 |
| 10 | 38.14 | 37.54 | 37.51 | 36.88 | 36.91 | 37.58 | 37.63 | 41.76 | 41.82 | 42.03 | 42.12 |
| 11 | 78.40 | 78.22 | 78.13 | 77.20 | 77.17 | 77.54 | 77.59 | 75.43 | 75.25 | 75.43 | 75.52 |
| 12 | 48.50 | 48.17 | 48.17 | 47.72 | 47.84 | 48.36 | 48.68 | 50.63 | 50.96 | 50.90 | 51.02 |
| 13 | 140.41 | 121.48 | 121.69 | 120.91 | 121.09 | 121.76 | 122.23 | 123.33 | 123.36 | 123.36 | 123.15 |
| 14 | 133.79 | 142.42 | 142.27 | 143.23 | 143.20 | 141.41 | 141.34 | 141.76 | 141.61 | 142.09 | 142.06 |
| 15 | 198.41 | 72.77 | 72.77 | 72.77 | 72.92 | 73.50 | 73.49 | 73.49 | 73.10 | 72.83 | 73.04 |
| 16 | 41.13 | 42.57 | 42.69 | 42.57 | 42.66 | 42.37 | 42.75 | 42.81 | 42.54 | 42.99 | 42.69 |
| 17 | 24.12 | 23.67 | 23.73 | 23.55 | 23.76 | 23.50 | 23.64 | 23.79 | 24.00 | 23.88 | 23.91 |
| 18 | 31.70 | 31.96 | 31.93 | 32.08 | 32.17 | 32.06 | 32.17 | 32.29 | 32.50 | 32.35 | 32.41 |
| 19 | 22,77 | 22.50 | 22.56 | 22.41 | 22.62 | 22.36 | 22.53 | 22.65 | 22.80 | 22.65 | 22.71 |
| 20 | 14.08 | 14.05 | 14.05 | 14.02 | 14.05 | 13.97 | 14.05 | 14.08 | 14.08 | 14.05 | 14.08 |
| $CH_3$ | - | 28.16 | 27.74 | 28.28 | 28.22 | 28.26 | 27.23 | 27.14 | 28.43 | 27.17 | 28.73 |

Tab. 4 $^{13}$C-chemische Verschiebungen a)

| C- | 3 | 4 | 4a | 5 | 5a | (15R)-5 c) | (15R)-5a | 10 | 10a | (15R)-10 | (15R)-10a |
|---|---|---|---|---|---|---|---|---|---|---|---|
| 1 | | | | | | | | 176.90 | 178.20 | 177.83 | 177.26 |
| 2 | | | | | | | | 32.56 | 33.11 | 33.13 | 32.98 |
| 3 | | | | | | | | 24.42 | 24.46 | 24.60 | 24.63 |
| 4 | | | | | | | | 26.33 | 26.37 | 26.60 | 26.36 |
| 55 | | | | | | | | 130.07 | 129.90 | 129.06 | 129.98 |
| 6 | 61.26 | 63.54 | 62.98 | 63.72 | 63.42 | 63.72 | 63.30 | 128.91 | 127.99 | 129.59 | 128.34 |
| 7 | 33.34 | 29.30 | 31.95 | 29.12 | 32.05 | 29.35 | 32.05 | 27.74 | 29.01 | 27.59 | 29.27 |
| 8 | 45.45 | 45.48 | 46.38 | 44.79 | 46.49 | 44.95 | 46.25 | 48.50 d) | 49.50 | 48.20 d) | 50.18 |
| 9 | 79.90 | 80.41 | 82.24 d) | 79.87 | 82.65 d) | 80.15 | 82.50 d) | 80.41 | 81.22 | 80.38 | 81.39 |
| 10 | 36.46 | 36.91 | 37.16 | 36.31 d) | 37.12 e) | 36.71 d) | 37.09 e) | 37.03 e) | 37.19 d) | 36.97 e) | 37.48 d) |
| 11 | 81.96 | 82.17 | 82.45 d) | 81.90 | 82.71 d) | 82.24 | 82.56 d) | 82.20 | 82.72 | 82.05 | 82.59 |
| 12 | 49.43 | 48.62 | 53.69 | 47.96 | 53.80 | 48.24 | 54.01 | 48.68 d) | 52.74 | 48.65 d) | 53.86 |
| 13 | 143.26 | 142.96 | 146.83 | b) | 132.17 | b) | 132.74 | b) | 132.42 | b) | 134.06 |
| 14 | 133.22 | 133.22 | 130.83 | 137.83 | 135.17 | 137.57 | 135.53 | 135.88 | 133.98 | 136.60 | 134.63 |
| 15 | 200.17 | 200.11 | 200.15 | 72.71 | 72.56 | 72.89 | 72.83 | 73.40 | 72.66 | 72.59 | 73.64 |
| 16 | 40.23 | 40.53 | 40.36 | 37.12 d) | 37.12 e) | 37.19 d) | 37.09 e) | 37.18 e) | 36.96 d) | 36.79 e) | 37.21 d) |
| 17 | 23.97 | 24.00 | 23.71 | 25.10 | 25.19 | 25.24 | 25.13 | 25.10 | 25.05 | 25.16 | 25.22 |
| 18 | 31.46 | 31.52 | 31.32 | 31.84 | 31.78 | 31.82 | 31.78 | 31.76 | 31.68 | 31.78 | 31.84 |
| 19 | 22.44 | 22.50 | 22.33 | 22.59 | 22.61 | 22.64 | 22.59 | 22.56 | 22.57 | 22.62 | 22.71 |
| 20 | 13.93 | 13.93 | 13.82 | 14.08 | 14.05 | 14.03 | 14.05 | 14.05 | 14.00 | 14.05 | 14.11 |
| $CH_2Ph$ | 71.18<br>72.05 | 71.30<br>71.90 | 71.43<br>71.76 | 70.94<br>71.90 | 71.60<br>71.72 | 70.99<br>71.86 | 71.69<br>71.54 | 70.94<br>71.63 | 71.25<br>71.64 | 70.94<br>71.66 | 71.30<br>71.87 |
| aromat. C | 138.04-<br>127.56 | 145.74-<br>127.11 | 145.51-<br>126.91 | 145.65-<br>126.36 | 145.62-<br>127.05 | 145.65-<br>126.92 | 145.62-<br>127.02 | 138.52-<br>127.48 | 138.47-<br>127.51 | 138.49-<br>126.99 | 139.64<br>127.59 |
| C=O | | 166.40 | 166.15 | 166.56 | 166.44 | 166.46 | 166.59 | | | | |

a) Wenn nichts anderes angegeben: 75.46 MHz, $CDCl_3$, TMS interner Standard

b) Signal durch Aromatensignale verdeckt

c) 20 MHz-Spektrum

d) e) Zuordnung in der vertikalen Spalte ist auch umgehrt möglich

0144459

## Patentansprüche

1. Prostaglandine des Typs 12-epi-$PGF_{2\beta}$ der allgemeinen Formel I,

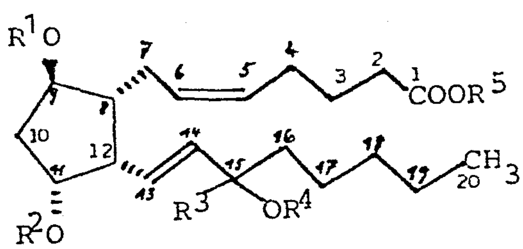

(I)

worin $R^1$ und $R^2$, die verschieden oder gleich sein können, ein Wasserstoffatom, eine Benzylgruppe, eine aliphatische Acylgruppe mit 2-5 C-Atomen, eine aromatische Acylgruppe mit 7-13 C-Atomen oder eine andere bekannte Schutzgruppe,

$R^3$ ein Wasserstoffatom, eine Tetrahydropyranylgruppe oder eine andere bekannte Schutzgruppe und

$R^5$ ein Wasserstoffatom, eine geradkettige oder verzweigte Alkylgruppe mit 1-4 C-Atomen oder eine bekannte Schutzgruppe

bedeuten, sowie deren physiologisch unbedenkliche Salze.

2. 15S-Epimere der Prostaglandine nach Anspruch 1.

3. 15R-Epimere der Prostaglandine nach Anspruch 1.

4. 9β,11α,15-Trihydroxy-5-cis,13-trans-12-epi-prostadiensäure und deren physiologisch unbedenkliche Salze.

5. 9β,11α,15S-Trihydroxy-5-cis,13-trans-12-epi-prostadiensäure und deren physiologisch unbedenkliche Salze.

6. 9β,11α,15R-Trihydroxy-5-cis,13-trans-12-epi-prostadiensäure und deren physiologisch unbedenkliche Salze.

7. 9β,11α,15-Trihydroxy-15-methyl-5-cis,13-trans-12-epi-prostadiensäure und deren physiologisch unbedenkliche Salze.

8. 9β,11α,15S-Trihydroxy-15-methyl-5-cis,13-trans-12-epi-prostadiensäure und deren physiologisch unbedenkliche Salze.

9. 9β,11α,15R-Trihydroxy-15-methyl-5-cis,13-trans-12-epi-prostadiensäure und deren physiologisch unbedenkliche Salze.

10. 9β,11α,15-Trihydroxy-5-cis,13-trans-12-epi-prostadiensäure-methylester.

11. 9β,11α,15S-Trihydroxy-5-cis,13-trans-12-epi-prostadiensäure-methylester.

12. 9β,11α,15R-Trihydroxy-5-cis,13-trans-12-epi-prostadiensäure-methylester.

13. 9β,11α,15-Trihydroxy-15-methyl-5-cis,13-trans-12-epi-prostadiensäure-methylester.

14. 9β,11α,15S-Trihydroxy-15-methyl-5-cis,13-trans-12-epi-prostadiensäure-methylester.

15. 9β,11α,15R-Trihydroxy-15-methyl-5-cis,13-trans-12-epi-prostadiensäure-methylester.

16. Verfahren zur Herstellung der Prostaglandine gemäß einem der Ansprüche 1 bis 15, gekennzeichnet durch die Kombination folgender Merkmale:

(a) eine Verbindung der allgemeinen Formel II,

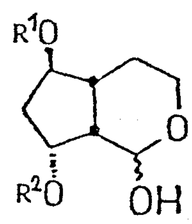

(II)

worin $R^1$ und $R^2$ die in Anspruch 1 genannten Bedeutungen, ausgenommen $R^1 = R^2 = H$, haben, wird mit einem geeigneten Wittig-Horner-Reagens zur entsprechenden Verbindung der allgemeinen Formel III,

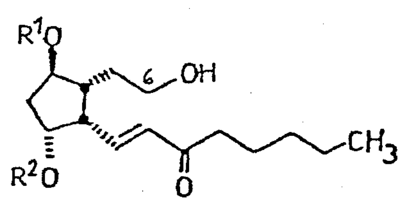

(III)

worin $R^1$ und $R^2$ die in Anspruch 1 genannten Bedeutungen, ausgenommen $R^1 = R^2 = H$, haben, umgesetzt;

(b) die 6-Hydroxygruppe der Verbindung der allgemeinen Formel III wird durch Einführung einer Schutzgruppe geschützt;

(c) die 15-Ketogruppe wird unter Bildung des 15S-/15R-Epimerengemischs der entsprechenden Verbindung der allgemeinen Formel IV,

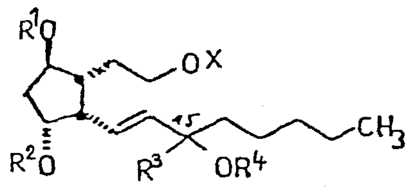

(IV)

worin $R^4$ ein Wasserstoffatom und X die Schutzgruppe ist, umgesetzt, und zwar für $R^3$ = H mit einem Reduktionsmittel und für $R^3$ = $C_1$- bis $C_3$-Alkyl mit einer metallorganischen Verbindung mit 1-3 C-Atomen, wonach die 15S- und 15R-Epimeren chromatographisch voneinander getrennt werden;

(d) die 15-Hydroxygruppe der Epimeren der allgemeinen Formel IV wird durch Einführung einer weiteren Schutzgruppe $R^4$ geschützt, wonach die Schutzgruppe X oxidativ abgespalten wird unter Bildung der entsprechenden Verbindung der allgemeinen Formel V,

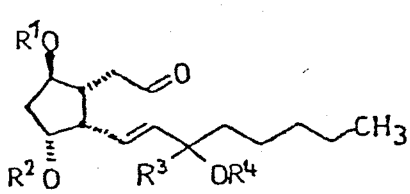

(V)

worin $R^1$, $R^2$, $R^3$ und $R^4$ die in Anspruch 1 genannten Bedeutungen, ausgenommen $R^1$, $R^2$, $R^4$ = H, haben;

(e) die Verbindung der allgemeinen Formel V wird mit einem geeigneten Pentansäure-Ylid zur entsprechenden Verbindung der allgemeinen Formel I umgesetzt, worin $R^1$, $R^2$, $R^3$ und $R^4$ die in Anspruch 1 genannten Bedeutungen, ausgenommen $R^1$, $R^2$, $R^4$ = H, haben und worin $R^5$ = H ist;

(f) wonach die Schutzgruppen $R^1$, $R^2$ und $R^4$ gegebenenfalls wieder abgespalten und/oder die freie Säure in ein physiologisch unbedenkliches Salz übergeführt oder verestert wird.

17. Verfahren nach Anspruch 16, dadurch gekennzeichnet, daß als Wittig-Horner-Reagens 2-Oxoheptylphosphonsäuredimethylester verwendet wird.

18. Verfahren nach Anspruch 16 oder 17, dadurch gekennzeichnet, daß als Schutzgruppe X der p-Phenylbenzoylrest verwendet wird.

19. Verfahren nach einem der Ansprüche 16 bis 18, dadurch gekennzeichnet, daß als metallorganische Verbindung mit 1-3 C-Atomen eine entsprechende Grignard-Verbindung verwendet wird.

20. Verfahren nach einem der Ansprüche 16 bis 19, dadurch gekennzeichnet, daß als Pentansäure-Ylid das aus 5-Triphenylphosphoniumpentansäurebromid und Kalium-tert.-butylat gebildete Ylid verwendet wird.

21. Verfahren zur Herstellung der Prostaglandine gemäß einem der Ansprüche 1 bis 15 und zur Herstellung von $PGF_{2\beta}$, gekennzeichnet durch die Kombination folgender Merkmale:

(a) eine Verbindung der allgemeinen Formel II, worin $R^1$ und $R^2$ die in Anspruch 1 genannten Bedeutungen, ausgenommen $R^1 = R^2 = H$, haben, wird mit einem geeigneten Reduktionsmittel zu einem Diol der allgemeinen Formel VII,

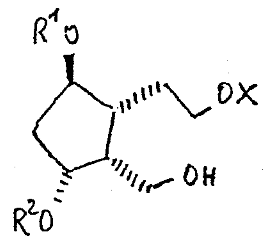

(VII)

worin $R^1$ und $R^2$ die in Anspruch 1 genannten Bedeutungen, ausgenommen $R^1 = R^2 = H$, haben und X = H ist, reduziert;

(b) das Diol wird mit einem reaktiven Säurederivat zur entsprechenden Verbindung der allgemeinen Formel VII, worin X der Acylrest des reaktiven Säurederivats ist, verestert;

(c) die verbliebene freie Hydroxymethylgruppe wird zur Aldehydgruppe unter Bildung der entsprechenden Verbindung der allgemeinen Formel VIII oxidiert;

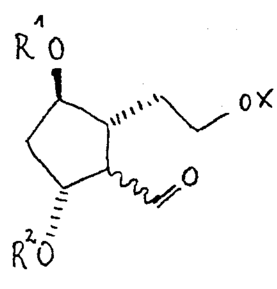

(VIII)

(d) die Verbindung der allgemeinen Formel VIII wird mit einem geeigneten Wittig-Horner-Reagens zum $C^{12}$-Epimerengemisch der allgemeinen Formel IX,

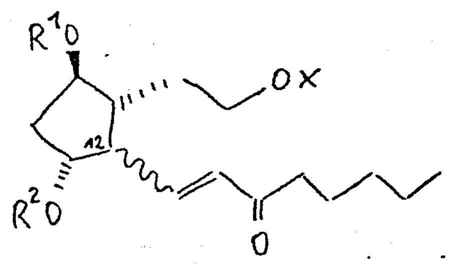

(IX)

worin $R^1$ und $R^2$ die in Anspruch 1 genannten Bedeutungen, ausgenommen $R^1 = R^2 = H$, haben und X die Acylschutzgruppe ist, umgesetzt, wonach die $C^{12}$-Epimeren säulenchromatographisch voneinander getrennt werden;

(e) das entsprechende 12-α-Epimere wird entsprechend Anspruch 16, Merkmale (a) bis (f), zu den entsprechenden Prostaglandinen der allgemeinen Formel I umgesetzt;

(f) das entsprechende 12β-Epimere wird unter Bildung des 15S/15R-Epimerengemischs der entsprechenden Verbindung der allgemeinen Formel X,

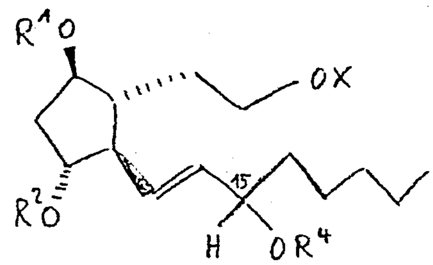

(X)

worin $R^4$ ein Wasserstoffatom ist, reduziert, wonach die $C^{15}$-Epimeren chromatographisch voneinander getrennt werden;

(g) die Verbindung der allgemeinen Formel X wird entsprechend Anspruch 16, Merkmale (d) bis (f), zum $PGF_{2\beta}$ (Formel VI) umgesetzt.

22. Verfahren nach Anspruch 21, dadurch gekennzeichnet, daß als Wittig-Horner-Reagens 2-Oxoheptylphosphonsäuredimethylester verwendet wird.

23. Verfahren nach Anspruch 21 oder 22, dadurch gekennzeichnet, daß als Acyl-Schutzgruppe X der p-Phenylbenzoylrest verwendet wird.

24. Verfahren nach einem der Ansprüche 21 bis 23, dadurch gekennzeichnet, daß als Pentansäure-Ylid das aus 5-Triphenylphosphoniumpentansäurebromid und Kalium-tert.-butylat gebildete Ylid verwendet wird.

25. Pharmazeutische Zubereitung, enthaltend eine Verbindung gemäß einem der Ansprüche 1 bis 15.

26. Pharmazeutische Zubereitung, enthaltend eine gemäß Anspruch 21 hergestellte Verbindung.

Europäisches Patentamt

# EUROPÄISCHER RECHERCHENBERICHT

0144459

Nummer der Anmeldung

EP 83 11 2409

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X | DE-A-2 360 893 (ICI)<br>* Ansprüche 1,6,13-15 *<br>--- | 1-15, 21-26 | C 07 C 177/00<br>A 61 K 31/557 |
| D,Y<br>X | DE-A-3 030 477 (FA. DR. WILLMAR SCHWABE)<br>* Ansprüche 1-9; Seite 8, Absatz 3 - Seite 12, Absatz 1 *<br>----- | 1-26 | |
| | | | **RECHERCHIERTE SACHGEBIETE (Int. Cl. 3)** |
| | | | C 07 C 177/00 |

Der vorliegende Recherchenbericht wurde für alle Patentansprüche erstellt.

| Recherchenort | Abschlußdatum der Recherche | Prüfer |
|---|---|---|
| DEN HAAG | 12-07-1984 | BERTE M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82